# EUROPÄISCHE PATENTSCHRIFT

(11) **EP 1 599 579 B1**
(45) Veröffentlichungstag und Bekanntmachung des Hinweises auf die Patenterteilung: **02.04.2008**
(21) Anmeldenummer: 04715290.5
(22) Anmeldetag: 27.02.2004
(51) Int. Cl.: C12N 1/21, C12N 1/20, C12N 15/31, C07K 14/195

(54) **EIN TRANSLOKATIONSENZYM ALS SELEKTIONSMARKER**
TRANSLOCATING ENZYME USED AS A SELECTION MARKER
ENZYME DE TRANSLOCATION SERVANT DE MARQUEUR DE SELECTION

(30) Priorität: 04.03.2003 DE 10309557
(43) Veröffentlichungstag der Anmeldung: 30.11.2005
(73) Patentinhaber: Henkel Kommanditgesellschaft auf Aktien, 40589 Düsseldorf (DE)
(72) Erfinder: HINTZ, Maren, 40468 Düsseldorf (DE); FREUDL, Roland, 52353 Düren (DE); FEESCHE, Jörg, 40699 Erkrath (DE); BREVES, Roland, 40822 Mettmann (DE)
(86) Internationale Anmeldenummer: PCT/EP2004/001949
(87) Internationale Veröffentlichungsnummer: WO 2004/078953

(56) Entgegenhaltungen:
- EP-A- 0 251 579
- EP-A- 0 894 857
- EP-A- 0 972 838
- WO-A-02/29113
- WO-A-96/26276
- WO-A-02/077183
- SADAIE Y ET AL: "SEQUENCING REVEALS SIMILARITY OF THE WILD-TYPE DIV-POSITIVE GENE OF BACILLUS-SUBTILIS TO THE ESCHERICHIA-COLI SEC-A GENE" GENE (AMSTERDAM), Bd. 98, Nr. 1, 1991, Seiten 101-105, XP002283176 ISSN: 0378-1119
- WELCH R A ET AL: "Extensive mosaic structure revealed by the complete genome sequence of uropathogenic Escherichia coli." PROCEEDINGS OF THE NATIONAL ACADEMY OF SCIENCES OF THE UNITED STATES, Bd. 99, Nr. 26, 24. Dezember 2002 (2002-12-24), Seiten 17020-17024, XP002283177 December 24, 2002 ISSN: 0027-8424 (ISSN print)

## Beschreibung

Die vorliegende Erfindung betrifft ein Selektionssystem für Mikroorganismen, welches auf der Inaktivierung eines essentiellen Translokationsenzyms und der Kurierung dieser Inaktivierung über einen gleichwirkenden Faktor beruht, welcher den betreffenden Zellen über einen Vektor zur Verfügung gestellt wird.

In großer Menge benötigte Proteine und hierunter insbesondere Enzyme für technische Einsatzgebiete werden heutzutage meist durch Fermentation von Mikroorganismen gewonnen. Gegenüber solchen Mikroorganismen, die die interessierenden Proteine natürlicherweise bilden, gewinnen gentechnisch modifizierte Produzenten-Stämme zunehmend an Bedeutung. Derartige gentechnische Verfahren zur Proteinherstellung sind im Stand der Technik seit langem etabliert. Das Prinzip hierfür besteht darin, daß die Gene für die interessierenden Proteine als Transgene in die Wirtszellen eingebracht, von diesen transkribiert, translatiert und gegebenenfalls durch die betreffenden Membranen in das Periplasma, beziehungsweise das umgebende Medium sekretiert werden. Sie sind dann aus den betreffenden Zellen beziehungsweise den Kulturüberständen erhältlich.

Bei technischen Verfahren zur Proteinherstellung werden zunächst die natürlichen Fähigkeiten der zur Produktion eingesetzten Mikroorganismen zur Synthese und gegebenenfalls zur Sekretion der Proteine ausgenutzt. Grundsätzlich werden als bakterielle Systeme zur Proteinherstellung solche ausgewählt, die kostengünstig in der Fermentation sind, die von vornherein eine hohe Produktbildungsrate versprechen und die eine korrekte Faltung, Modifizierung etc. des herzustellenden Proteins gewährleisten; letzteres ist mit zunehmender Verwandtschaft mit dem das interessierende Protein ursprünglich produzierenden Organismus umso wahrscheinlicher. Besonders zu diesem Zweck etablierte Wirtszellen sind gramnegative Bakterien, wie beispielsweise *Escherichia coli* oder *Klebsiella,* oder grampositive Bakterien, wie beispielsweise Spezies der Gattungen *Staphylococcus* oder *Bacillus.*

Die Wirtschaftlichkeit eines biotechnologischen Verfahrens hängt entscheidend von der erzielbaren Ausbeute an Protein ab. Diese Ausbeute wird neben dem verwendeten Expressionssystem vom eingesetzten Verfahren, insbesondere über die Fermentationsparameter und die Substrate bestimmt. Durch Optimierung des Expressionssystems und des Fermentationsprozesses können das Potential eines Produktionsorganismus und die erreichbare Ausbeute deutlich gesteigert werden.

Darüber hinaus werden Expressionssysteme im wesentlichen auf der Basis von zwei grundsätzlich verschiedenen genetischen Konstruktionen entwickelt und weiterentwickelt. Einerseits wird das Gen für das herzustellende Protein in das Chromosom des Wirtsorganismus integriert. Derartige Konstrukte sind ohne Selektion auf Anwesenheit eines zusätzlichen Markergens (siehe unten) sehr stabil. Der Nachteil ist, daß nur eine Kopie des Gens im Wirt vorhanden ist und sich die Integration weiterer Kopien zur Erhöhung der Produktbildungsrate über den Gendosiseffekt methodisch sehr aufwendig gestaltet. Dieser Stand der Technik sei im folgenden kurz illustriert.

Das europäische Patent EP 284126 B1 löst das Problem einer stabilen Mehrfachintegration darüber, daß mehrere Genkopien in die Zelle eingebracht werden, die dazwischenliegende, endogene und essentielle chromosomale DNA-Abschnitte enthalten.

Eine andere Lösung zur stabilen Mehrfachintegration wird in der Patentanmeldung WO 99/41358 A1 offenbart. Demnach werden zwei Kopien des interessierenden Gens in entgegengesetzten Transkriptionsrichtungen integriert und von einem nicht-essentiellen DNA-Abschnitt voneinander getrennt, um somit homologe Rekombination der beiden Kopien zu verhindern.

Aus der Patentanmeldung DD 277467 A1 geht ein Verfahren zur Herstellung von extrazellulären Enzymen hervor, das auf der stabilen, vorteilhafterweise mehrfachen Integration der für das interessierende Enzym codierenden Gene in das Bakterienchromosom beruht. Die Integration erfolgt über homologe Bereiche. Zur Kontrolle erfolgreicher Integrationsereignisse dient ein auf dem Plasmid enthaltenes Erythromycin-Gen, das bei erfolgreicher Integration inaktiviert wird.

Nach der Schrift DE 4231764 A1 kann die Integration ins Chromosom über ein einfaches oder doppeltes Crossing-over über das Gen für die Thymidylat-Synthetase erfolgen. Letzteres erlaubt die Kontrolle dieses Vorgehens, denn bei einem einfachen Crossing-over bleibt die *thy*-Aktivität erhalten, während sie bei einem doppelten Crossing-over verloren geht, das heißt hierdurch eine Auxotrophie erzielt wird. Mit einem einfachen Crossing-over geht in diesem speziellen System eine Resistenz gegen das Antibiotikum Trimethoprim einher, mit einem doppelten eine entsprechende Sensitivität.

In der Anmeldung WO 96/23073 A1 wird ein Transposon-basiertes System zur Integration von mehrfachen Kopien eines interessierenden Gens in das Bakterienchromosom offenbart, das dadurch gekennzeichnet ist, daß das Marker-Gen des Plasmids durch die Integration deletiert wird und die erhaltenen Stämme somit frei von einem Resistenzmarker sind. Auch nach dieser Schrift wird ein Marker nur für die Steuerung der Konstruktion des betreffenden Bakterienstamms benötigt.

Ein System zur Erhöhung der Kopienzahl von bestimmten, in ein bakterielles Chromosom integrieren Transgenen wird auch in der Anmeldung WO 01/90393 A1 offenbart.

Der zweite Ansatz zur Konstruktion von Produzenten-Stämmen besteht darin, das interessierende Gen auf ein autonom replizierendes Element, zum Beispiel ein Plasmid zu übertragen und auf diese Weise in den Wirtsorganismus zu transferieren. Vorteilhaft wirkt sich dabei über den Gendosis-Effekt die üblicherweise hohe Zahl von Plasmidkopien pro Zelle aus. Nachteilig ist die Tatsache, daß über die gesamte Kulturzeit hinweg ein Selektionsdruck ausgeübt werden muß, um die Plasmide in den Zellen zu halten. Standardmäßig erfolgt dies über den Zusatz von Antibiotika in das Kulturmedium, während Gene, die gegen die betreffenden Substanzen Resistenz verleihen, auf den Plasmiden vorgelegt werden. Damit vermögen lediglich die Zellen zu wachsen, welche die Plasmide in ausreichender Zahl besitzen.

Der Einsatz von Antibiotikaresistenzen als Selektionsmarker stößt in den letzten Jahren zunehmend auf Kritik. Zum einen ist der Einsatz von Antibiotika recht teuer, insbesondere, wenn die Resistenz auf einem das Antibiotikum abbauenden Enzym beruht und die betreffende Substanz deshalb während der gesamten Kultivierung zugeführt werden muß. Zum anderen trägt ihr weitverbreiteter Einsatz, insbesondere auf anderen Technik-Gebieten zur Ausbreitung der Resistenzgene auf andere Stämme, sogar auf pathologische Stämme bei. Dies führt beispielsweise in der medizinischen Hygiene und insbesondere bei der Behandlung von Infektionskrankheiten bereits zu erheblichen Schwierigkeiten durch sogenannte multiresistente humanpathogene Stämme.

Deshalb wird in großem Maße auf die oben illustrierten Systeme zur stabilen Integration von Genen ins Chromosom der Produzenten-Zellen zurückgegriffen, weil diese ohne einen permanenten Selektionsdruck stabil sind. Allerdings sind die betreffenden Stämme, wie oben erwähnt nur unter großem Aufand herstellbar. Schneller und komfortabler ist es in der biotechnologischen Praxis, ein beispielsweise neugefundenes oder modifiziertes Gen auf einem Plasmid mit Selektionsmarker in Wirtszellen einzubringen und auf diese Weise zu exprimieren.

Im Stand der Technik sind inzwischen auch Antibiotika-freie Selektionssysteme entwickelt worden. So werden beispielsweise in der Publikation "Transposon vectors containing non-antibiotic resistance selection markers for cloning and stable chromosomal insertion of foreign genes in gram-negative bacteria" von Herrero et al. (1990), in J. Bacteriol., Band 172, Seiten 6557-6567, Resistenzen gegen Herbizide und Schwermetalle als Selektionsmarker beschrieben. Gegen den Einsatz dieser Verbindungen sprechen jedoch dieselben Bedenken wie gegen Antibiotika.

Prinzipiell ähnlich wie eine Antibiotikum-Selektion funktioniert beispielsweise die Selektion über Auxotrophie, das heißt über einen gezielten Stoffwechseldefekt, der die betreffenden Zellen von der Zufuhr bestimmter Stoffwechselprodukte abhängig macht. Auxotrophe Stämme erhalten dann gekoppelt mit dem interessierenden Transgen eines, das diese Auxotrophie kuriert. Bei Verlust würden sie unter entsprechenden Kulturbedingungen gleichzeitig ihre Lebensfähigkeit verlieren, so daß es zur erwünschten Selektion der auxotrophen Produzenten-Stämme kommt. So wird beispielsweise in der Publikation "Gene cloning in lactic streptococci" von de Vos in Netherlands Milk and Dairy Journal, Band 40, (1986), Seite 141-154 auf S. 148 auf verschiedene, aus dem Stoffwechsel von Lacto-Streptococci entwickelte Selektionsmarker verwiesen; hierunter sind solche aus dem Lactose-Stoffwechsel, Kupfer-Resistenz und Resistenzgene gegenüber verschiedenen Bacteriocinen von Lacto-Streptococci. Das Patent EP 284126 B1, das sich mit dem Problem der stabilen Integration interessierender Gene ins Bakterienchromosom befaßt (siehe oben) faßt die zur Selektion möglichen Systeme Auxotrophie, Resistenz gegen Biozide und Resistenz gegen Virus-Infektionen auf S. 7 unter dem Begriff "Überlebens-Selektion" zusammen. Als Beispiele für Auxotrophie-Selektionsmarker werden hier die Stoffwechselgene *leu*, *his, trp* "oder ähnliche" angeführt; gemeint sind offensichtlich solche aus Aminosäuresynthesewegen.

In der Praxis gestaltet sich der Einsatz solcher Auxotrophien aber bisher sehr problematisch, da insbesondere in industriellen Fermentationsmedien fast alle notwendigen Substrate in ausreichenden Mengen zur Verfügung stehen und die betreffenden Zellen den Mangel zur Synthese einer bestimmten Verbindung über die Aufnahme ebendieser Verbindung aus dem Nährmedium ausgleichen können.

Eine Ausnahme ist bisher nur das essentielle Thymidin, das in industriellen Fermentationsmedien lediglich in Spuren vorkommt und deshalb von den - proliferierenden und somit DNA-synthetisierenden - Organismen über eine Thymidylat-synthase gebildet werden muß. So bietet die Anmeldung EP 251579 A2 die Lösung an, als Wirts-Stämme solche einzusetzen, die hinsichtlich des für den Nukleotid-Stoffwechsel essentielen Gens für die Thymidylat-Synthase defizient sind. Über einen Vektor kann demnach das Gen für eben diese Funktion (*thyA* aus *Escherichia coli* K12) zur Verfügung gestellt werden und den Gen-Defekt kurieren. Trägt dieser Vektor zusätzlich das Gen für das interessierende Protein, so kommt es zu einer Antibiotikumähnlichen Selektion der Produzenten-Zellen.

Zusammenfassend muß man feststellen, daß im Stand der Technik zwar große Erfahrung hinsichtlich der biotechnologischen Produktion von Proteinen besteht und die Expression interessierender Gene über chromosomale Integration und Antibiotika-Selektion gezielt steuerbar ist, zu diesen beiden Systemen bislang jedoch so gut wie keine praktikablen Alternativen bestehen, insbesondere keine, die weniger aufwendig sind als die chromosomale Integration und gleichzeitig ohne Selektion über eine teure oder ökologisch bedenkliche Verbindung auskommen. Insbesondere der Ansatz zur Selektion über Auxotrophie-Marker hat bislang hinsichtlich der in der Industrie allgemein üblichen komplexen Nährmedien nur zu sehr spärlichen Ergebnissen geführt.

Es stellte sich somit die Aufgabe, ein neues Selektionssystem zu entwickeln, das im vergleichbar einfach zu handhaben ist wie die Selektion über ein Antibiotikum, jedoch ohne teure und unter Umständen umweltschädliche Substanzen auskommt. Es sollte in industriellem Maßstab anwendbar sein. Es sollte nicht auf einem essentiellen Gen aufbauen, dessen Fehlen über Begleitstoffe in industriellen Medien ausgeglichen werden kann.

Diese Aufgabe wird erfindungsgemäß durch Verfahren zur Selektion eines Mikroorganismus gelöst, die dadurch gekennzeichnet sind, daß
(a) ein endogen vorhandenes für eine essentielle Translokations-Aktivität codierendes Gen inaktiviert und
(b) die nach (a) inaktivierte essentielle Translokations-Aktivität über einen Vektor kuriert wird.

Überraschenderweise wurde erkannt, daß die an der Translokation beteiligten essentiellen Faktoren für eine Selektion geeignet sind. Zur Selektion dient erfindungsgemäß ein Gen, dessen abgeleitetes Protein als ein für die betreffende Zelle essentieller Faktor an der Protein-Translokation beteiligt ist. Das bedeutet, daß der Ausfall dieses Gens letal ist und damit eine Antibiotikum-ähnliche Selektion von Mikroorganismen möglich ist.

Dieses Selektionssystem kommt ohne Zusatzstoffe (wie etwa die im Stand der Technik etablierten Antibiotika) aus und funktioniert prinzipiell unabhängig von der Zusammensetzung der Nährmedien. Dafür ist eine molekularbiologische Modifikation der betreffenden Mikroorganismen notwendig, die im Anschluß an die Ausführungen zur Translokation beschrieben und in den Beispielen der vorliegenden Anmeldung veranschaulicht wird.

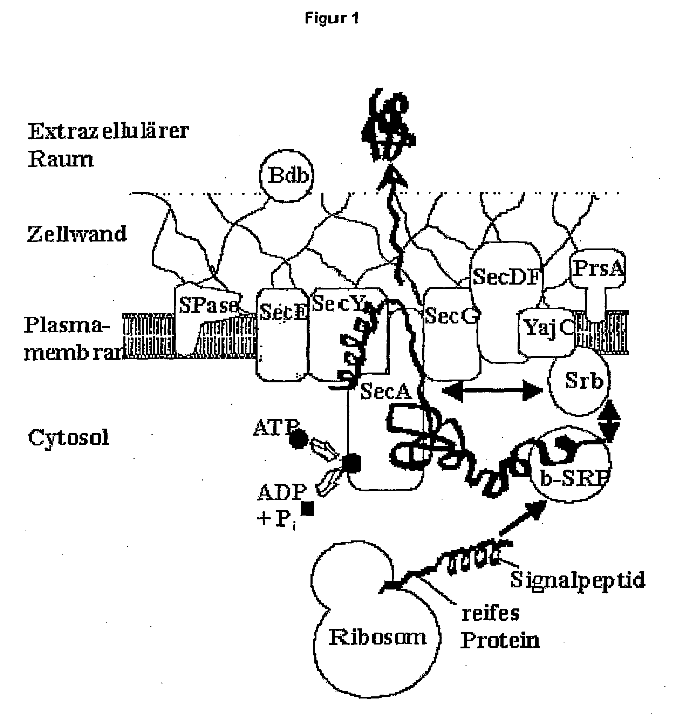

Bei dem Prozeß der Translokation handelt es sich um die Sekretion der von Bakterien gebildeten Proteine in das Periplasma (bei gramnegativen Bakterien), beziehungsweise das umgebende Medium (sowohl bei gramnegativen als auch bei grampositiven Bakterien). Sie ist beispielsweise in dem Aufsatz von A.J. Driessen (1994): "How proteins cross the bacterial cytoplasmic membrane" in J. Membr. Biol., 142 (2), S. 145-59 beschrieben. Der dazu notwendige Sekretionsapparat besteht aus einer Reihe unterschiedlicher, hauptsächlich membranassoziierter Proteine, die in Figur 1 der vorliegenden Anmeldung gezeigt sind. Hierzu gehören insbesondere die beispielsweise für *Bacillus subtilis* gut charakterisierten Proteine SecA, SecD, SecF (zusammen als Komplex SecDF), E, G und Y (van Wely, K.H., Swaving, J., Freudl, R., Driessen, A.J. (2001): "Translocation of proteins across the cell envelope of Gram-positive bacteria", FEMS Microbiol Rev. 2001, 25(4), S. 437-54.). Weitere diesem System zuzurechnende Faktoren sind YajC, der mit dem Sec-Komplex ebenfalls in direkten Kontakt tritt, und die ebenfalls in Figur 1 zu erkennenden Faktoren Bdb (Dsb), SPase (für "Signalpeptidase"), PrsA und b-SRP (Ffh, Ffs/Scr, SRP-RNA).

Bei dem zuletzt genannten Faktor handelt es sich um den bakteriellen Faktor, der prinzipiell dieselbe Funktion wie das ursprünglich in Eukaryonten beschriebene SRP (signal recognition particle) übernimmt. Eine Untereinheit davon ist der Faktor Ffh, der sowohl aus *B. subtilis* als auch aus *E. coli* charakterisiert ist. Die nächste Untereinheit von b-SRP heißt in *B. subtilis* Scr und in *E. coli* Ffs. Ferner ist eine RNA (SRP-RNA) Teil des funktionellen b-SRP-Komplexes. Ein weiterer funktionell hiermit vergesellschafteter Faktor heißt in *E. coli* Srb und in *B. subtilis* FtsY. Er entspricht funktionell dem eukaryontischen Docking Protein.

In einem weiteren Kontext ist beispielsweise auch der Faktor PrfB (peptide chain release factor B; auch RF2) einzubeziehen. Er ist als Teil des Apparats zur Proteinsynthese bekannt, und zwar sowohl bei grampositiven als auch bei gramnegativen Bakterien. Er ist im Zusammenhang mit der Translation insbesondere für die Ablösung der fertig translatierten Proteine vom Ribosom, das heißt für die Termination der Translation verantwortlich. Der Zusammenhang zur oben dargestellten Translokation ist nur indirekt darüber gegeben, daß das Gen *prfB* in vielen Bakterien gleichzeitig mit dem Gen für den Faktor SecA transkribiert wird. Es besteht also ein regulatorischer Zusammenhang.

Voraussetzung für die Translokation ist, daß die auszuschleusenden Proteine N-terminal über ein Signalpeptid verfügen (Park, S., Liu, G., Topping, T.B., Cover, W.H., Randall, L.L. (1988): "Modulation of folding pathways of exported proteins by the leader sequence", Science, 239, S. 1033-5). Dies gilt sowohl für extrazelluläre als auch für Membranproteine.

Nach der an den Ribosomen erfolgten Translation wird die entstandene Peptidkette durch cytoplasmatische Proteine mit Chaperon-Funktion in einem ungefalteten Zustand gehalten und zur Membran transportiert. Dann wird unter ATP-Verbrauch der Transport des Peptids durch die Membran katalysiert (Mitchell, C., Oliver, D. (1993): "Two distinct ATP-binding domains are needed to promote protein export by Escherichia coli SecA ATPase", Mol. Microbiol., 10(3), S. 483-97). SecA fungiert dabei als die energieliefernde Komponente (ATPase) des Multienzymkomplexes Translokase. Nach Überwindung der Membran wird das Signalpeptid wird durch die Aktivität einer Signalpeptidase abgespalten und das extrazelluläre Protein auf diese Weise von der Membran abgelöst. Bei grampositiven Bakterien erfolgt auf diese Weise die Ausschleusung der Exoproteine direkt ins umgebende Medium. Bei gramnegativen Bakterien befinden sich die Proteine anschließend in der Regel im Periplasma und es bedarf weiterer Modifikationen, um ihre Freisetzung in das umgebende Medium zu erreichen.

Eine neuere Untersuchung von B.v.d.Berg et al. (2004; Nature, Band 427, Seiten 36 bis 44) beschreibt die Röntgenstruktur der Translokase, das heißt des SecY-Komplexes aus *Methanococcus jannaschii* als Modell für den entsprechenden Komplex in anderen Organismen.

Zentrales Molekül dieses Prozesses ist somit die aus den Untereinheiten SecA, SecY, SecE, SecD, SecF (SecDF) und SecG bestehende Präprotein-Translokase. Als der diesen Prozeß kontrollierenden ATPase kommt dem Faktor SecA dabei eine entscheidende Funktion für die Translokation zu. Durch diese Faktoren sind die bevorzugten Ausführungsformen der vorliegenden Erfindung gekennzeichnet (siehe unten).

In der folgenden Tabelle 1 sind die bislang bekannten, an der Translokation beteiligten Faktoren aufgeführt und dahingehend aufgeschlüsselt, ob sie in einem der beiden Modellorganismen *Escherichia coli* (gramnegativ) und *Bacillus subtilis* (grampositiv) essentiell sind. Sofern einer davon im jeweils betrachteten Organismus essentiell ist, eignet er sich zur erfindungsgemäßen Selektion. Grundsätzlich ist davon auszugehen, daß ein entsprechendes Muster auch in anderen Organismen, insbesondere gramnegativen und grampositiven Bakterien besteht.

**Tabelle 1: An der Translokation von gramnegativen und grampositiven Bakterien beteiligte, bislang bekannte Proteinfaktoren, aufgeschlüsseit danach, ob sie in diesen Organismen essentiell sind.**

| | ***E. coli*** | ***B. subtilis*** |
|---|---|---|
| **SecA** | essentiell | essentiell |
| **SecY** | essentiell | essentiell |
| **SecE** | essentiell | essentiell |
| **SecG** | nicht essentiell | nicht essentiell |
| | (kältesensitiver Phänotyp) | (kältesensitiver Phänotyp bei Überproduktion von Exportproteinen) |
| **SecD, SecF** | essentiell | nicht essentiell |
| **(SecDF)** | | (kältesensitiver Phänotyp) |
| **Signalpeptidase** | essentiell | nicht essentiell, |
| | | da redundant vorhanden |
| **b-SRP (Ffh; Ffs / Scr; SRP-RNA)** | essentiell | essentiell |
| **FtsY / Srb** | essentiell | essentiell |
| **PrsA** | nicht vorhanden | essentiell |
| **Bdb / Dsb** | nicht essentiell | nicht essentiell |
| **YajC** | essentiell | nicht bekannt, ob essentiell, jedoch redundant vorhanden |

Erfindungsgemäß kann somit in gramnegativen Bakterien, insbesondere in coliformen Bakterien, ganz besonders in *E. coli* über folgende Transloaktionsenzyme beziehungsweise deren zugehörige Gene selektiert werden: SecA, SecY, SecE, SecD, SecF, Signalpeptidase, b-SRP (Ffh oder Ffs), Srb oder YajC.

Erfindungsgemäß kann somit in grampositiven Bakterien, insbesondere in *Bacillus,* ganz besonders in *B. subtilis* über folgende Transloaktionsenzyme beziehungsweise deren zugehörige Gene selektiert werden: SecA, SecY, SecE, b-SRP (Ffh oder Scr), FtsY oder PrsA.

Die Oder-Verknüpfung in diesen Aufzählungen ist nicht ausschließend zu verstehen. Technisch sollte es möglich sein, auch mehrere der zugehörigen Gene gleichzeitig auszuschalten. Erfindungsgemäß ist es jedoch ausreichend, lediglich eine dafür auszuwählen.

Jeweils einzelne Sequenzen der zugehörigen Gene sind beispielsweise aus folgenden allgemein zugänglichen Datenbanken erhältlich: GenBank (National Center for Biotechnology Information NCBI, National Institutes of Health, Bethesda, MD, USA; http://www3.ncbi.nlm.nih.gov); EMBL-European Bioinformatics Institute (EBI) in Cambridge, Großbritannien (http://www.ebi.ac.uk); Swiss-Prot (Geneva Bioinformatics (GeneBio) S.A., Genf, Schweiz; http://www.genebio.com/sprot.html); "Subtilist" oder "Colibri" des Institut Pasteur, 25,28 rue du Docteur Roux, 75724 Paris CEDEX 15, Frankreich für Gene und Faktoren aus *B. subtilis* beziehungsweise *E. coli* (http://genolist.pasteur.fr/SubtiList/ beziehungsweise http://genolist.pasteur.fr/Colibri/). Ferner kann auf die Datenbanken zurückgegriffen werden, die über Querverweise in den genannten Datenbanken erreichbar sind. Erfindungsgemäß ist es jeweils nur notwendig, ein einziges essentielles Gen des Translokationsapparats in dem für die Kultivierung beabsichtigten Stamm zu identifizieren und entsprechend zu verwenden.

Einen weiteren Ansatzpunkt liefern die im Sequenzprotokoll zur vorliegenden Anmeldung angegebenen Sequenzen für den Faktor SecA aus verschiedenen Mikroorganismen. Diese können entweder direkt (siehe unten: bevorzugte Ausführungsformen) verwendet werden oder eingesetzt werden, um das betreffende Homolog in einer Genbank zu identifizieren, die man zuvor für den interessierenden Mikroorganismus angelegt hat.

Unter diesen Translokationsenzymen oder -Faktoren sind in erster Linie die Wildtypmoleküle zu verstehen. Sofern es jedoch möglich ist, Varianten davon herzustellen, die im Translokationsapparat über prinzipiell dieselbe Funktion wie das Wildtypenzym verfügen, so werden hierauf aufbauende Selektionssysteme in den Schutzbereich miteingeschlossen.

Zur Realisierung der vorliegenden Erfindung muß zunächst der für die Kultivierung beabsichtigte Stamm, sofern er nicht einer der in der vorliegenden Anmeldung untersuchten Spezies angehört, dahingehend überprüft werden, ob zumindest einer dieser Faktoren essentiell ist. Dies ist auf einfache Weise beispielsweise dadurch möglich, daß eines dieser bekannten Gene aus einem möglichst verwandten Stamm (beispielsweise einem ebenfalls gramnegativen beziehungsweise grampositiven Bakterium) entnommen oder anhand eines Eintrags in einer allgemein zugänglichen Datenbank synthetisiert und in einen Knock-out-Vektor eingebracht wird. Ein derartiges Vorgehen ist dem Fachmann allgemein bekannt. Wirken sich die Transformation mit diesem Vektor und eine anschließende - vorzugsweise getrennt von der Transformation eingeleitete - homologe Rekombination dieses Gens in das Genom der Wirtszelle letal aus, so ist das betrachtete Gen als essentiell anzusehen. Dieses als essentiell erkannte Gen kann nun erfindungsgemäß und insbesondere nach dem Vorbild der Beispiele der vorliegenden Anmeldung als Selektionsmarker eingesetzt werden.

Eine nach Merkmal (a) erforderliche Inaktivierung erfolgt beispielsweise über homologe Rekombination einer inaktivierten Genkopie, die beispielsweise durch Transformation mit einem entsprechenden Vektor in eine Zelle des interessierenden Mikroorganismus-Stammes eingebracht worden ist. Methoden hierzu sind an sich bekannt. Als Ergebnis des Rekombinationsereignisses wird die chromosomale Kopie des Gens ganz oder teilweise deletiert und damit funktionsunfähig. Dies kann beispielsweise über dasselbe Gen erfolgen, mit welchem zuvor der Test auf Letalität durchgeführt worden ist. Vorzugsweise wird jedoch, sofern bekannt oder mit vertretbarem Aufwand zu isolieren, das endogene Homolog eingesetzt, um für die Rekombination eine hohe Erfolgsquote zu erzielen. Ausschlaggebend ist für die Verwirklichung der Erfindung, ob die Inaktivierung erfolgreich ist.

Dieses Konzept läßt sich beispielsweise mit Plasmidvektoren realisieren, die einen temperatursensitiven Replikationsursprung besitzen und in die zusätzlich die in Frage kommenden homologen DNA-Breiche des zu deletierenden Gens eingefügt wurden (Deletionsvektor). Denkbar wäre beispielsweise auch eine reversible Inaktivierung, beispielsweise über Integration eines mobilen genetischen Elements, zum Beispiel eines Transposons, in das Zielgen.

Hierbei ist jeweils Merkmal (b) zu beachten, nämlich daß bereits vor diesem Rekombinations- beziehungsweise Inaktivierungsereignis oder spätestens gleichzeitig eine intakte Kopie des zur erfindungsgemäßen Selektion ausgewählten Gens in der betreffenden Zelle bereitgestellt wird, weil die Zelle die Inaktivierung sonst nicht überleben würde. Erfindungsgemäß wird der entstandene Defekt über einen Vektor ausgeglichen, das heißt der Vektor kuriert die Inaktivierung. Hierbei werden wie oben ausgeführt vorzugsweise die endogen in den Wirtszellen vorhandenen und gemäß (a) deletierten Gene verwendet. Es können aber auch funktionsgleiche Gene aus anderen Organismen, vorzugsweise verwandten Stämmen eingesetzt werden, sofern sie in der Lage sind, den betreffenden Defekt zu kurieren. So ist es beispielsweise möglich, den Defekt von SecA eines *B. subtilis* durch Bereitstellung eines *secA*-Gens aus *Staphylococcus camosus* zu kurieren.

Denkbar wäre auch, daß über den Vektor ein anderes, den ersten Defekt aufhebendes genetisches Element in die Zelle gebracht wird, beispielsweise das Gen eines funktionell prinzipiell gleichen, jedoch durch Mutation veränderten Faktors.

In dieser Zelle herrscht somit die Situation, daß ein letaler Defekt über ein separates genetisches Element ausgeglichen wird. Ein Verlust dieses separaten genetischen Elements wäre wiederum letal, so daß eine solche Zelle gezwungen ist, bei jeder Zellteilung dieses Element an die Folgegeneration weiterzugeben.

Dies macht sich in einer bevorzugten Ausführungsform (siehe unten) Merkmal (c) zunutze, wonach der den Gendefekt ausgleichende Vektor das Transgen, eben das Gen für das interessierende Protein trägt, welches über das erfindungsgemäße Verfahren hergestellt werden soll.

Es herrscht gewissermaßen ein endogener Selektionsdruck, ohne daß von außen, das heißt über das Nährmedium die Gabe einer sonstigen Verbindung, etwa eines Schwermetalls oder eines Antibiotikums nötig wäre, um den Verlust des Vektors mit dem Tansgen zu verhindern. Andererseits entfallen die eingangs diskutierten, aufwendigen Modifikationen, um das Transgen selbst in die chromosomale DNA zu integrieren. So kann beispielsweise ein einmal erzeugter Mikroorganismen-Stamm, der zu einer definierten Inaktivierung des Translokationsapparates vorbereitet ist, für immer neue Transformationen mit ähnlich aufgebauten Vektoren verwendet werden, die jedesmal dieselbe den Gendefekt kurierende Funktion zur Verfügung stellen, aber jeweils verschiedene Transgene tragen. Man verfügt somit über ein sehr praktisches und vielseitig einsetzbares Selektionssystem.

Der Sinn des erfindungsgemäßen Selektionsverfahrens besteht darin, ein genetisches Element über mehrere Generationen hinweg stabil in der Zelle zu erhalten. Dieses Element ist eben der Vektor, über den die Inaktivierung der essentiellen Translokations-Aktivität ausgeglichen, das heißt kuriert wird.

In einer bevorzugten Ausführungsform sind erfindungsgemäße Selektionsverfahren dadurch gekennzeichnet, daß
(c) der Vektor nach (b) ein Transgen trägt.

Denn dies ist das technisch wichtigste Einsatzgebiet von Selektionssystemen. Das über mehrere Generationen hinweg stabile genetische Element ist dann eines, das ein Transgen trägt, insbesondere eines, dessen Genprodukt von kommerziellem Interesse ist. Bevorzugte Ausführungsformen hiervon werden weiter unten ausgeführt.

In bevorzugten Ausführungsformen ist ein erfindungsgemäßes Selektionsverfahren dadurch gekennzeichnet, daß es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

Denn wie in Tabelle 1 zusammengestellt hadelt es sich bei diesen Faktoren beziehungsweise den zugehörigen Genen um solche, von denen entweder aus *E. coli* oder aus *B. subtilis* bekannt ist, daß sie essntiell sind. Es liegt daher auf der Hand, insbesondere in diesen beiden Organismen aber auch in verwandten oder sogar weniger verwandten Spezies ein erfindungsgemäßes Selektionssystem über die betreffenden Homologe zu etablieren. Da bekannt ist, daß sogar einzelne dieser Gene die betreffende Funktion im jeweils anderen Organismus hinweg substituieren können, das heißt über die Grenze gramnegativ/grampositiv hinweg, sollten zumindest einzelne der betreffenden Gene sogar aus nur entfernt verwandten Spezies erfindungsgemäß einsetzbar sein.

Vorzugsweise handelt es sich bei der essentiellen Translokations-Aktivität um eine der folgenden Untereinheiten der Präprotein-Translokase: SecA, SecY, SecE, SecD oder SecF, vorzugsweise um die Untereinheit SecA.

Denn diese Faktoren stellen, wie in Figur 1 gezeigt ist, gewissermaßen den funktionellen Kern des Translokationsapparats dar. Zu SecA ist weiter oben ausgeführt worden, daß dieser Faktor über die ATPase-Aktivität eine wichtige Schlüsselstellung einnimmt. Aus diesem Grund ist die Erfindung auch in den Beispielen zur vorliegenden Anmeldung anhand des Gens *secA* umgesetzt worden.

Vorzugsweise sind erfindungsgemäße Selektionsverfahren dadurch gekennzeichnet, daß das Kurieren nach (b) über eine zu der inaktivierten endogen vorhandenen essentiellen Translokations-Aktivität gleichwirkende Aktivität, vorzugsweise über eine genetisch verwandte, besonders bevorzugt über dieselbe Aktivität erfolgt.

Darin spiegelt sich wieder, daß aufgrund der allgemein hohen Homologiewerte zwischen den Spezies für die betreffenden Faktoren auch die betreffenden Gene aus weniger verwandten Spezies erfolgversprechend eingesetzt werden können. Bevorzugt sind aber selbstverständlich solche aus näher verwandten Spezies und ganz besonders aus denselben Organismen, weil diese hinsichtlich des notwendigen Crossing-over zur Inaktivierung am erfolgversprechendsten sind. Es sei nochmal darauf hingeweisen, daß lediglich ein einziges zur Inaktivierung geeignetes Gen ausreicht, um eine erfindungsgemäße Selektion zu realisieren.

Wie oben bereits gesagt, sind die betreffenden DNA- und Aminosäuresequenzen aus allgemein zugänglichen Datenbanken erhältlich. So wurden beispielsweise die im Sequenzprotokoll unter SEQ ID NO. 1 und 2 angegebenen Sequenzen für das Protein SecA aus *B. subtilis* aus der Datenbank "Subtilist" des Institut Pasteur (siehe oben) entnommen (Stand: 3.2.2003); sie sind mit der von Swiss-Prot (siehe oben) identisch, die dort unter der Zugangsnummer P28366 hinterlegt sind.

Die im Sequenzprotokoll unter SEQ ID NO. 3 und 4 angegebenen Sequenzen für das Protein SecA aus *E. coli* stammen aus der Datenbank "Colibri" des Institut Pasteur (siehe oben; Stand: 3.2.2003); sie sind mit der von Swiss-Prot (siehe oben) identisch, die dort unter der Zugangsnummer P10408 abgerufen werden können.

SEQ ID NO. 5 und 6 für *B. licheniformis* wurden wie in Beispiel 1 zur vorliegenden Anmeldung beschrieben aus dem kommerziell erhältlichen Stamm *B. licheniformis* (DSM13) erhalten (Deutsche Sammlung von Mikroorganismen und Zellkulturen GmbH, MascheroderWeg 1b, 38124 Braunschweig; http://www.dsmz.de).

Weil es sich bei den Spezies *Bacillus subtilis, Escherichia coli* und *Bacillus licheniformis* um die technisch am häufigsten eingesetzten Mikroorganismen handelt, ist es besonders wichtig, ein erfindungsgemäßes Verfahren für diese Bakterien zur Verfügung zu stellen. Mit dem Sequenzprotokoll zur vorliegenden Anmeldung werden die homologen *secA-*Gene aus diesen drei wichtigsten Organismen zur Verfügung gestellt, ohne daß sie für die Nacharbeitung isoliert werden müßten. Über diese Gene ist es beispielweise möglich, die betreffenden Homologen in anderen Mikroorganismen zu identifizieren, etwa über Herstellung einer Genbank und Screening mit einem dieser Gene als Sonde. Insbesondere in verwandten Spezies können aber auch diese Gene selbst für eine erfindungsgemäße Inaktivierung eingesetzt werden.

Bevorzugte Ausführungsformen sind somit dadurch gekennzeichnet, daß das Kurieren nach (b) über die die Translokationsaktivität wiederherstellenden Bereiche des Gens *secA* aus *Bacillus subtilis, Escherichia coli* oder *Bacillus licheniformis* erfolgt, die im Sequenzprotokoll unter SEQ ID NO. 1, SEQ ID NO. 3 beziehungsweise SEQ ID NO. 5 angegeben sind.

Bevorzugte Verfahren sind darüberhinaus dadurch gekennzeichnet, daß die Inaktivierung nach (a) so erfolgt, daß eine Rekombination zwischen dem nach (a) inaktivierten Genbereich und dem homologen Bereich auf dem Vektor nach (b) verhindert wird, vorzugsweise unter vollständigem Verlust eines in dem betreffenden chromosomalen Gen enthaltenen Genabschnitts.

Denn wenn der Vektor in das Chromosom der Wirtszelle integrieren würde, wäre die letale Mutation dauerhaft kuriert, ohne daß gleichzeitig ein Selektionsdruck auf den betreffenden Vektor besteht. Dadurch könnte über die nachfolgenden Zellteilungen das eigentlich interessierende Transgen verloren gehen. Weitgehende Deletion während des Inaktivierungsschritts (a) verhindert dies.

Im Stand der Technik, insbesondere in der Publikation "Genetic manipulation of Bacillus amyloliquefaciens"von J.Vehmaanperä et al. (1991) in J. Biotechnol., Band 19, Seiten 221-240, sind Verfahren zur Inaktivierung von Genen über einen Deletionsvektor beschrieben. Anhand dieser Beschreibung konnte in Beispiel 3 die Deletion des Gens *secA* von *B. licheniformis* erfolgreich durchgeführt werden. Der Replikationsursprung dieses Deletionsvektors zeichnet sich durch seine Temperatur-Abhängigkeit aus.

Dadurch ist es besonders leicht möglich, zunächst bei niedrigerer Temperatur auf eine erfolgreiche Transformation zu selektieren und anschließend durch Temperaturerhöhung einen Selektionsdruck auf eine erfolgreiche Integration, das heißt Inaktivierung des endogenen Gens auszuüben. Analog wäre beispielsweise auch eine Konstruktion zur Steuerung über die Zugabe von niedermolekularen Verbindungen möglich.

Bevorzugte erfindungsgemäße Verfahren sind folglich dadurch gekennzeichnet, daß die Inaktivierung nach (a) über einen Deletionsvektor erfolgt, vorzugsweise über einen Deletionsvektor mit einem extern regulierbaren Replikationsursprung, besonders bevorzugt über einen Deletionsvektor mit einem Temperatur-abhängigen Replikationsursprung.

Wie oben ausgeführt, ist es prinzipiell möglich, daß sich der nach (b) kurierende Vektor, einschließlich des Transgens ins bakterielle Chromosom integriert. Da hierbei jedoch die Gefahr besteht, daß das Transgen dabei auf Dauer verloren geht, sind bevorzugte Verfahren dadurch gekennzeichnet, daß es sich bei dem Vektor nach (b) um ein in dem Mikroorganismus autonom replizierendes Plasmid handelt. Dieses etabliert sich dann in der abgeleiteten Zellinie.

Besonders vorteilhaft ist es, wenn es sich bei dem Plasmid um eines sich in mehrfacher (beispielsweise 2 bis 100 Plasmide pro Zelle), vorzugsweise in vielfacher Kopienzahl (mehr als 100 Plasmide pro Zelle) etablierendes Plasmid handelt. Denn je mehr Plasmidkopien vorliegen, desto erfolgreicher dürfte die Kurierung sein. Außerdem steigert dies in dem Fall, daß der Vektor ein für ein interessierendes Protein codierendes Transgen trägt, über den Gendosiseffekt die erzielbare Ausbeute an diesem Produkt.

Aufgrund der großen Bedeutung gramnegativer Bakterien-Stämme, insbesondere bei der Klonierung und Charakterisierung von Genen beziehungsweise Genprodukten sind bevorzugte Selektionsverfahren dadurch gekennzeichnet, daß es sich bei dem Mikroorganismus um einen gramnegativen Bakterien-Stamm handelt.

Hierunter sind insbesondere Verfahren zu verstehen, die dadurch gekennzeichnet sind, daß es sich um einen gramnegativen Bakterien-Stamm der Gattungen *E. coli* oder *Klebsiella* handelt, insbesondere um Derivate von *Escherichia coli* K12, von *Escherichia coli* B oder *Klebsiella planticola,* und ganz besonders um Derivate der Stämme *Escherichia coli* BL21 (DE3), *E. coli* RV308, *E. coli* DH5α, *E.coli* JM109, *E. coli* XL-1 oder *Klebsiella planticola* (Rf). Denn dieses sind die in der Molekularbiologie am häufigsten eingesetzten Organismen.

Insbesondere für die fermentative Proteinherstellung sind grampositive Bakterien von besonderer Bedeutung. Dies gilt in besonderem Maße für sekretierte Proteine. Bevorzugte erfindungsgemäße Verfahren sind deshalb dadurch gekennzeichnet, daß es sich bei dem Mikroorganismus um einen grampositiven Bakterien-Stamm handelt.

Hierunter sind insbesondere in der Technik grampositive Bakterien-Stämme der Gattungen *Staphylococcus, Corynebakterien* oder *Bacillus* etabliert, insbesondere der Spezies *Staphylococcus carnosus, Corynebacterium glutamicum, Bacillus subtilis, B. licheniformis, B. amyloliquefaciens, B. globigii* oder *B. lentus,* und ganz besonders Derivate der Stämme *B. licheniformis* oder *B. amyloliquefaciens*, weshalb diese entsprechend bevorzugte Selektionsverfahren kennzeichnen.

Von ganz besonderem Interesse sind erfindungsgemäße Verfahren, die auf bestimmte, durch die Kultivierung der Mikroorganismen hergestellte Produkte ausgerichtet sind. Entsprechend bevorzugt sind somit Selektionsverfahren, die dadurch gekennzeichnet sind, daß es sich bei dem Transgen nach (c) um eines handelt, das für ein Nicht-Enzym-Protein codiert, insbesondere für ein pharmakologisch relevantes Protein, ganz besonders für Insulin oder Calcitonin.

Aber auch Enzyme sind von großer technischer Bedeutung. Also werden erfindungsgemäß auch solche Verfahren beansprucht, die dadurch gekennzeichnet sind, daß es sich bei dem Transgen nach (c) um eines handelt, das für ein Enzym codiert, vorzugsweise für ein hydrolytisches Enzym oder eine Oxidoreduktase, besonders bevorzugt für eine Protease, Amylase, Hemicellulase, Cellulase, Lipase, Cutinase, Oxidase, Peroxidase oder Laccase. Hiervon seien die für den Einsatz in Wasch- und Reinigungsmitteln bevorzugt produzierten Vertreter im folgenden ausgeführt.

Prinzipiell können derartigen Mitteln zur Steigerung der Wasch-, beziehungsweise Reinigungsleistung alle im Stand der Technik etablierten Enzyme eingesetzt werden. Unter den Proteasen sind solche vom Subtilisin-Typ bevorzugt, beispielsweise die Alkalische Protease aus *Bacillus lentus*. Von der Protease aus *Bacillus lentus* DSM 5483 (WO 91/02792 A1) leiten sich die unter der Bezeichnung BLAP® geführten Varianten ab, die insbesondere in WO 92/21760 A1, WO 95/23221 A1, WO 02/088340 A2 und WO 03/038082 A2 beschrieben werden. Weitere erfindungsgemäß herstellbaren Proteasen aus verschiedenen *Bacillus sp*. und *B. gibsonii* gehen aus den Patentanmeldungen WO 03/054185 A1, WO 03/056017 A2, WO 03/055974 A2 und WO 03/054184 A1 hervor.

Beispiele für erfindungsgemäß herstellbare Amylasen sind die α-Amylasen aus *Bacillus licheniformis,* aus *B. amyloliquefaciens* oder aus *B. stearothermophilus* sowie deren, insbesondere für den Einsatz in Wasch- und Reinigungsmitteln verbesserte Weiterentwicklungen. Desweiteren sind für diesen Zweck die in der Anmeldung WO 02/10356 A2 offenbarte α-Amylase aus *Bacillus sp*. A 7-7 (DSM 12368) und die in der Anmeldung WO 02/44350 A2 beschriebene Cyclodextrin-Glucanotransferase (CGTase) aus *B. agaradherens* (DSM 9948) hervorzuheben. Ferner liegen die amylolytischen Enzyme im Blickfeld der vorliegenden Anmeldung, die dem Sequenzraum von α-Amylasen angehören, der in der Anmeldung WO 03/002711 A2 definiert wird, und die, die in der Anmeldung WO 03/054177 A2 beschrieben werden. Ebenso sind Fusionsprodukte der genannten Moleküle gemeint, beispielsweise die aus der Anmeldung DE 10138753 A1.

Erfindungsgemäß können auch Lipasen oder Cutinasen hergestellt werden, beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L oder die Lipasen beziehungsweise Cutinasen, deren Ausgangsenzyme ursprünglich aus *Pseudomonas mendocina* und *Fusarium solanii* isoliert worden sind.

Ferner ist insbesondere für die technische Behandlung von Textilien, aber auch für Waschmittel an Cellulasen gedacht, beispielsweise Endoglucanase, und/oder Cellobiohydrolasen. Beispielsweise auch durch die natürlichen Produzenten herstellbare Cellulasen sind die aus *Bacillus sp.* CBS 670.93 und CBS 669.93, wie sie in WO 96/34092 A2 offenbart werden. Ferner können erfindungsgemäß weitere Enzyme hergestellt werden, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen.

Ebenfalls zu den Wasch- und Reinigungsmittel-Enzymen zählen Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) oder alle anderen im Stand der Technik für dieses Einsatzgebiet beschriebenen Enzyme.

Wie bereits einleitend ausgeführt, stellte sich die der vorliegenden Erfindung zugrundeliegende Aufgabe besonders deutlich vor dem Hintergrund der großtechnischen Fermentation. Denn gerade dort zeigten sich die Nachteile, daß einerseits eine Selektion über Antibiotika teuer und unter Umweltschutzgesichtspunkten problematisch ist, andererseits eine auf Stoffwechseldefekten beruhende Auxotrophie aufgrund der Komplexität industrieller Medien ausgeglichen werden kann.

Von besonderer Bedeutung ist daher die Umsetzung eines erfindungsgemäßen Selektionsverfahrens in großtechnischen Verfahren, etwa zur Herstellung niedermolekularer Verbindungen wie Antibiotika oder Vitamine oder ganz besonders zur Proteinherstellung.

Verfahren zur Herstellung eines Proteins durch Kultivierung von Zellen eines Mikroorganismus-Stammes sind im Stand der Technik allgemein bekannt. Sie beruhen darauf, daß die Zellen, die das interessierende Protein natürlicherweise oder nach Transformation mit dem betreffenden Gen produzieren, auf geeignete Weise in Kultur genommen und vozugsweise zur Bildung des interessierenden Proteins angeregt werden.

Einen weiteren Erfindungsgegenstand bilden somit Verfahren zur Herstellung eines Proteins durch Kultivierung von Zellen eines Mikroorganismus-Stammes, die durch ein zuvor beschriebenes Selektionsverfahren gekennzeichnet sind. Hierauf heben insbesondere die weiter oben bereits dargestellten Ausführungsformen ab, daß der kurierende Vektor selbst ein Transgen enthält und daß dieses bevorzugt für ein Nicht-Enzym-Protein oder für ein Enzym codiert. Hierunter sind insbesondere kommerziell bedeutende Proteine zu verstehen. So wird transgen hergestelltes Insulin zur Behandlung der Diabetes eingesetzt und ein breites Spektrum an Enzymen, von Proteasen, Lipasen und Amylasen bis hin zu oxidativen Enzymen zur Herstellung von Wasch- und Reinigungsmitteln.

Aufgrund der einfachen technischen Handhabbarkeit, wenn das interessierende Transgen und das kurierende Gen auf einem Vektor bereitgestellt werden, sind solche erfindungsgemäßen Proteinherstellungsverfahren bevorzugt, die dadurch gekennzeichnet sind, daß das Transgen nach (c) für das über dieses Verfahren hergestellte Protein codiert.

Prinzipiell können Bakterien auf einer festen Oberfläche herangezogen werden. Dies ist insbesondere zum Testen ihrer Stoffwechseleigenschaften oder zur dauerhaften Kultivierung im Labormaßstab von Bedeutung. Für die Produktion von Proteinen sind demgegenüber Verfahren bevorzugt, die dadurch gekennzeichnet sind, daß die Kultivierung der Mikroorganismen in einem flüssigen Medium erfolgt, vorzugsweise in einem Fermenter. Derartige Techniken sind im Stand der Technik weit verbreitet und werden erfindungsgemäß eben durch die Umstellung auf eine Selektion über essentielle Translokationsfaktoren weiterentwickelt.

Von besonderer Bedeutung sind Proteinherstellungsverfahren, die dadurch gekennzeichnet sind, daß das interessierende Protein ins umgebende Medium sekretiert wird. Denn hierdurch wird die Aufarbeitung des erhaltenen Produkts wesentlich erleichtert. Eine erfindungsgemäß mögliche Alternative besteht jedoch auch darin, die betreffenden das Protein herstellenden Zellen im Anschluß an die eigentliche Produktion aufzuschließen und dadurch das Produkt zu rhalten.

Prinzipiell wird durch jede molekularbiologische Veränderung an einem Mikroorganismus ein neuer Stamm erzeugt. Somit werden auch durch Umsetzung der vorliegenden Erfindung neue Mikroorganismen-Stämme hergestellt, eben solche, die sich vom Ausgangsstamm (genauer gesagt: von der Ausgangszelle) durch die gezielte Inaktivierung einer essentiellen Translokationsaktivität und dessen Kurierung durch Bereitstellung eines gleichwirkenden Translokationsfaktors unterscheiden. Durch die Anwendung eines erfindungsgemäßen Selektionsverfahrens werden also neuartige Mikroorganismen hergestellt.

In den Schutzbereich der vorliegenden Anmeldung fallen somit Mikroorganismen, die durch ein zuvor beschriebenes Selektionsverfahren erhalten worden sind.

Ein besonders vorteilhafter Aspekt besteht darin, daß eine Schar verwandter Mikroorganismen dadurch erhalten wird, daß immer dieselbe Art der Inaktivierung und Kurierung durchgeführt, auf dem kurierenden Vektor aber jedesmal ein anderes Transgen bereitgestellt wird. Ein einmal erfolgreich angewendetes Verfahren kann auf diese Weise auf unzählige weitere Selektionsprobleme übertragen werden.

Zur Realisierung insbesondere der oben ausgeführten Proteinherstellungsverfahren ist es notwendig, daß das Transgen exprimiert wird. Hierdurch sind erfindungsgemäß bevorzugte Mikroorganismen gekennzeichnet.

Hierunter sind entsprechend dem oben zur Proteingewinnung Gesagten die Mikroorganismen bevorzugt, die dadurch gekennzeichnet sind, daß das Transgen sekretiert wird.

Entsprechend dem oben Gesagten konnten erfindungsgemäße Selektionssysteme nur dadurch gefunden werden, daß essentielle, für Translokations-Aktivitäten codierende Gene als solche erkannt wurden, über die eine Selektion möglich ist. Eine derartige Verwendung dieser Gene ist bislang noch nicht angedacht worden, obgleich zahlreiche davon aus einer Vielzahl von Mikroorganismen bekannt sind. Gerade diese Bekanntheit kommt erfindungsgemäßen Selektionsystemen zugute, denn somit können praktisch für alle Mikroorganismen Gene definiert werden, über die eine entsprechende Selektion möglich ist. Sie müssen dafür wie oben ausgeführt lediglich inaktiviert und in der betreffenden Zelle durch ein funktionierendes Homologes substituiert werden.

Einen Erfindungsgegenstand bildet somit die Verwendung eines für eine essentielle Translokations-Aktivität codierenden Gens zur Selektion eines Mikroorganismus, die dadurch gekennzeichnet ist, daß
(a) diese in dem Mikroorganismus endogen vorhandene essentielle Translokations-Aktivität inaktiviert wird und
(b) die nach (a) inaktivierte essentielle Translokations-Aktivität über einen Vektor kuriert wird.

Die im folgenden aufgeführten Ausführungsformen dieses Erfindungsgegenstands sind entsprechend den bisherigen Darstellungen entsprechend bevorzugt.

Von besonderem molekulargenetischen und technischen Interesse ist es, wenn diese Verwendung dadurch gekennzeichnet ist, daß
(c) der Vektor nach (b) ein Transgen trägt.

Einen Schwerpunkt der vorliegenden Erfindung bildet jede entsprechende Verwendung die dadurch gekennzeichnet ist, daß es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

Hierunter ist jede Verwendung bevorzugt, die auf der essentiellen Translokations-Aktivität einer der folgenden Untereinheiten der Präprotein-Translokase beruht: SecA, SecY, SecE, SecD oder SecF, vorzugsweise die Untereinheit SecA.

Eine vorteilhafte Verwendung besteht darin, daß das Kurieren nach (b) über eine zu der inaktivierten endogen vorhandenen essentiellen Translokations-Aktivität gleichwirkende Aktivität, vorzugsweise über eine genetisch verwandte, besonders bevorzugt über dieselbe Aktivität erfolgt.

Hierfür stellt die vorliegende Anmeldung einige entsprechend bevorzugte Ansatzpunkte zur Verfügung, nämlich die genannten Verwendungsmöglichkeiten, die insbesondere dadurch gekennzeichnet sind, daß das Kurieren nach (b) über die die Translokationsaktivität wiederherstellenden Bereiche des Gens *secA* aus *Bacillus subtilis, Escherichia coli* oder *Bacillus licheniformis* erfolgt, die im Sequenzprotokoll unter SEQ ID NO. 1, SEQ ID NO. 3 beziehungsweise SEQ ID NO. 5 angegeben sind.

Besonders vorteilhafte Verwendungen sind dadurch gekennzeichnet, daß es sich bei dem Vektor nach (b) um ein in dem Mikroorganismus autonom replizierendes Plasmid handelt.

Weiter bevorzugte Verwendungen sind dadurch gekennzeichnet, daß es sich bei dem Plasmid um eines sich in mehrfacher, vorzugsweise in vielfacher Kopienzahl etablierendes Plasmid handelt.

Schließlich wird die vorliegende Erfindung auch durch die Bereitstellung entsprechender Vektoren verwirklicht. Gemeint sind hiermit Vektoren, die ein Gen für eine essentielle Translokations-Aktivität und ein expressionsfähiges Transgen tragen, welches allerdings, wenn es als einziges Transgen vorhanden ist, nicht für eine Antibiotikum-Resistenz codiert.

Der Auschluß von Genen für eine Antibiotikum-Resistenz für den Fall, daß es sich dabei um das einzige Transgen neben dem die erfindungsgemäß inaktivierte essentielle Translokations-Aktivität kurierenden Gen handelt, ist im Sinne der vorliegenden Anmeldung nicht technich bedingt. Er berücksichtigt lediglich, daß im Stand der Technik im Zusammenhang mit der Charakterisierung der erfindungsgemäß verwendbaren Translokationsproteine eben diese bereits beschrieben sind. Dies beinhaltet selbstverständlich auch, daß sie sequenziert und kloniert worden sind und zwar über die im Stand der Technik am weitesten verbreiteten Klonierungsvektoren, eben solche, die als Marker Gene für eine Antibiotikumsresistenz enthalten. Somit sind Vektoren, die lediglich durch ein Gen für ein essentielles Translokationsenzym und einen Antibiotikummarker enthalten, im Stand der Technik bekannt.

Entsprechend dem oben Gesagten ist ein erfindungsgemäßer Vektor vorzugsweise dadurch gekennzeichnet, daß das auf ihm enthaltene, vorzugsweise für die Proteinherstellung gedachte Transgen für ein pharmakologisch relevantes Nicht-Enzym-Protein oder für ein hydrolytisches Enzym oder für eine Oxidoreduktase codiert. Solche sind dadurch gekennzeichnet, daß das zu exprimierende Gen mit einem funktionierenden Promotor versehen ist. Hier werden selbstverständlich alle solche Konstrukte in den Schutzbereich eingeschlossen, die auch für - eventuell pharmakologisch interessante - Faktoren codieren, die eine Antibiotikumsresistenz vermitteln können, sofern auf die Anwesenheit dieses Vektors nicht über diese Eigenschaft sondern über die essentielle Translokationsaktivität selektiert wird.

Entsprechend den Ausführungen zum Selektionssystem stellen auch bestimmte Vektoren bevorzugte Ausführungsformen der vorliegenden Erfindung dar.

Hierzu gehören entsprechende Vektoren die dadurch gekennzeichnet sind, daß die von ihnen codierte Translokations-Aktivität eine in einem Mikroorganismenstamm endogen vorhandene, inaktivierte essentielle Translokations-Aktivität zu kurieren vermag, vorzugsweise über eine genetisch verwandte, besonders bevorzugt über dieselbe Aktivität.

Weiter bevorzugt gehören hierzu entsprechende Vektoren die dadurch gekennzeichnet sind, daß es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

Hierunter sind die Vektoren bevorzugt, die dadurch gekennzeichnet sind, daß es sich bei der essentiellen Translokations-Aktivität um eine der folgenden Untereinheiten der Präprotein-Translokase handelt: SecA, SecY, SecE, SecD oder SecF, vorzugsweise um die Untereinheit SecA.

Entsprechend der Lehre der vorliegenden Anmeldung sind weiterhin die Vektoren bevorzugt die dadurch gekennzeichnet sind, daß es sich bei der essentiellen Translokations-Aktivität um eines der Gene *secA* aus *Bacillus subtilis, Escherichia coli* oder *Bacillus licheniformis* handelt, die im Sequenzprotokoll unter SEQ ID NO. 1, SEQ ID NO. 3 beziehungsweise SEQ ID NO. 5 angegeben sind.

Ferner handelt es sich um eine günstige Eigenschaft der erfindungsgemäßen Vektoren wenn es sich bei ihnen um in dem verwendeten Mikroorganismus autonom replizierende Plasmide handelt.

Hierbei ist es insbesondere wegen des Gendosiseffekts besonders vorteilhaft, wenn die Plasmide dadurch gekennzeichnet sind, daß es sich dabei um sich in mehrfacher, vorzugsweise in vielfacher Kopienzahl etablierende Plasmide handelt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", *Cold Spring Harbour Laboratory* Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

### Isolierung des Gens secA aus B. licheniformis

### Identifizierung des secA-Locus in B. licheniformis

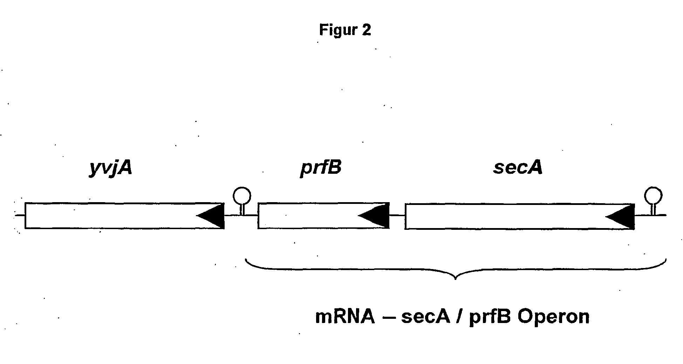

Zur Identifizierung des *secA*/*prfB*-Locus in *B. licheniformis* wurde über PCR eine Gensonde anhand der bekannten Sequenz des *prfB-secA*-Genorts von *B. subtilis* (Datenbank "Subtilist" des Institut Pasteur, 25,28 rue du Docteur Roux, 75724 Paris CEDEX 15, Frankreich; http://genolist.pasteur.fr/SubtiList/; Stand: 16.8.2002) abgeleitet. Dieser Genort ist auch in Figur 2 dargestellt. Die erhaltene Sonde war 3113 bp lang und umfaßte zusätzlich die ersten 451 bp des N-terminalen Bereichs des Gens prfB. Anschließend wurden Präparationen von chromosomaler DNA von *B. licheniformis,* der beispielsweise von der Deutschen Sammlung von Mikroorganismen und Zellkulturen GmbH, Mascheroder Weg 1b, 38124 Braunschweig (http://www.dsmz.de) unter der Bestellnummer 13 erhältlich ist, und zur Kontrolle chromosomale DNA von *B. subtilis* mit verschiedenen Restriktionsenzymen verdaut und einer Southern-Hybridisierung mit der genannten Sonde unterworfen. An der mit dem Restriktionsenzym *Mun*I behandelten chromosomalen DNA von *B. licheniformis* wurde ein einziges Fragment in der Größe von ca. 5,5 kB identifiziert, während der Verdau der chromosomalen DNA von *B. subtilis* mit *Mun*I die für *B. subtilis* erwarteten Fragmente lieferte.

### Klonierung des identifizierten Bereich aus B. licheniformis DSM13

Chromosomale DNA desselben Stamms *B. licheniformis* wurde isoliert, präparativ mit *Mun*I verdaut und der DNA-Bereich um 5,5 kB über Agarosegel-Elektrophorese isoliert sowie die Nucleinsäuren daraus mit kommerziell erhältlichen Kits extrahiert. Das erhaltene Gemisch von *Mun*I-geschnittenen DNA-Fragmenten wurde in die mit *Mun*I kompatible *Eco*RI-Schnittstelle des Low-copy-number-Vektors pHSG575 (beschrieben in: "High-copy-number and low-copy-number vectors for lacZ alpha-complementation and chloramphenicol- or kanamycin-resistance selection"; S. Takeshita; M. Sato; M. Toba; W. Masahashi; T. Hashimoto-Gotoh; Gene (1987), Band 61, Seiten 63-74) ligiert und in *E. coli* JM109 (erhältlich von der Firma Promega, Mannheim, Deutschland) transformiert.

Es wurde auf die vom Vektor kodierte Resistenz selektiert. Zusätzlich diente die Methode des Blau/Weiß-Screenings (Selektionsplatten enthielten 80 µg/ml X-Gal) zur Identifizierung von Klonen, die einen Vektor mit Insert aufgenommen hatten. Darüber wurden 200 Klone erhalten, von denen über Koloniehybridisierung 5 Klone identifiziert werden konnten, die das *B. licheniformis-secA*-Gen trugen. Diese wurden durch erneut Southern-Blot-Analyse mit der oben beschriebenen Sonde überprüft und ein von pHSG575 abgeleiteter Vektor mit dem das *secA*-Gen von *B. licheniformis* tragenden, 5,5 kB umfassenden *Mun*I Fragment unter der Bezeichnung pHMH1 fortgeführt.

### Restriktionsanalyse

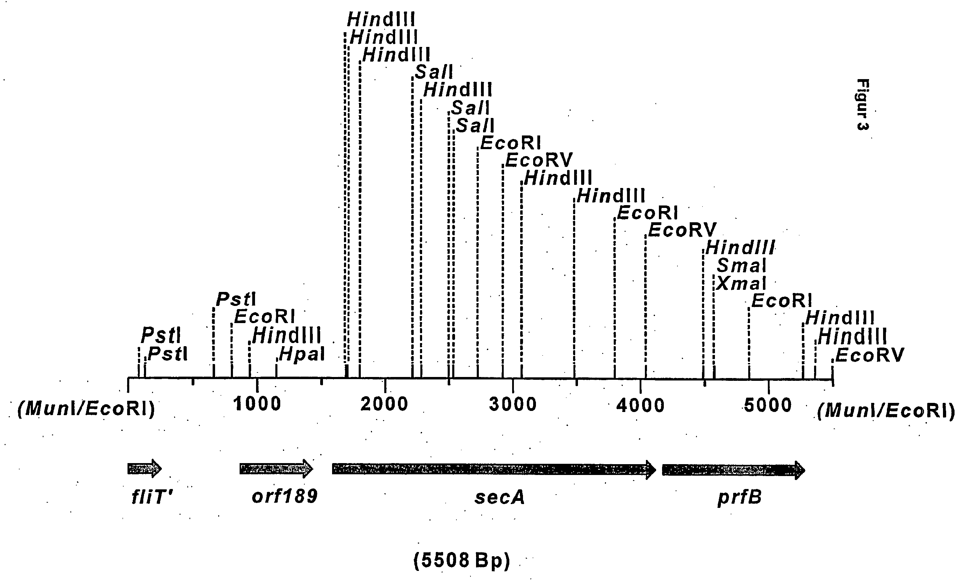

Der klonierte 5,5 kB-Bereich wurde zunächst über Restriktionskartierung charakterisiert. Hierzu wurden mit verschiedenen Enzymen Einzel- und Doppelverdaus von pHMH1 durchgeführt und mittels Southern-Blot-Analyse solche Fragmente identifiziert, die Teile des *secA*/*prfB*-Operons tragen. Die daraus resultierende Restriktionskarte wurde nach vollständiger Sequenzierung des 5,5 kB Fragments (siehe unten) ergänzt und ist in Figur 3 zu sehen.

### Sequenzanalyse

Das in Figur 3 gezeigte 5,5 kB große Fragment wurde in Teilsequenzen nach StandardMethoden sequenziert. Die Teilsequenzen zeigten starke Homologien zu folgenden Genen aus *B. subtilis: fliT* (codiert für ein Flagellen-Protein), *orf189*/*yvyD* (unbekannte Funktion), *secA* (Translokase-bindende Untereinheit; ATPase) und *prfB* (Peptide Chain Release Factor 2), in genau der gleichen Genabfolge wie bei *B. subtilis.* Diese sind ebenfalls in Figur 3 gezeigt.

Aufgrund dieser Werte kann auch davon ausgegangen werden, daß das SecA aus *B. licheniformis* dieselbe biochemische Aktivität wie insbesondere das SecA von *B. subtilis* ausübt und damit dieselbe physiologische Funktion übernimmt. Es ist somit als essentielles, in die Translokation eingebundenes Enzym zu betrachten.

Die nach diesem Beispiel ermittelte DNA- und die davon abgeleitete Aminosäuresequenz sind im Sequenzprotokoll unter SEQ ID NO. 5 beziehungsweise 6 angeführt. Demnach liegt der Translationsstart an der Position 154 und das Stop-Codon in den Positionen 2677 bis 2679. Als Promotor-Bereich ist vermutlich die Teilsequenz von den Positionen 60 bis 65 beziehungsweise 77 bis 82 anzusehen und als Ribosomen-Bindungsstelle der Bereich von Position 138 bis 144.

### Beispiel 2

### Herstellung eines Plasmids mit secA-Gen und Subtilisin-Gen

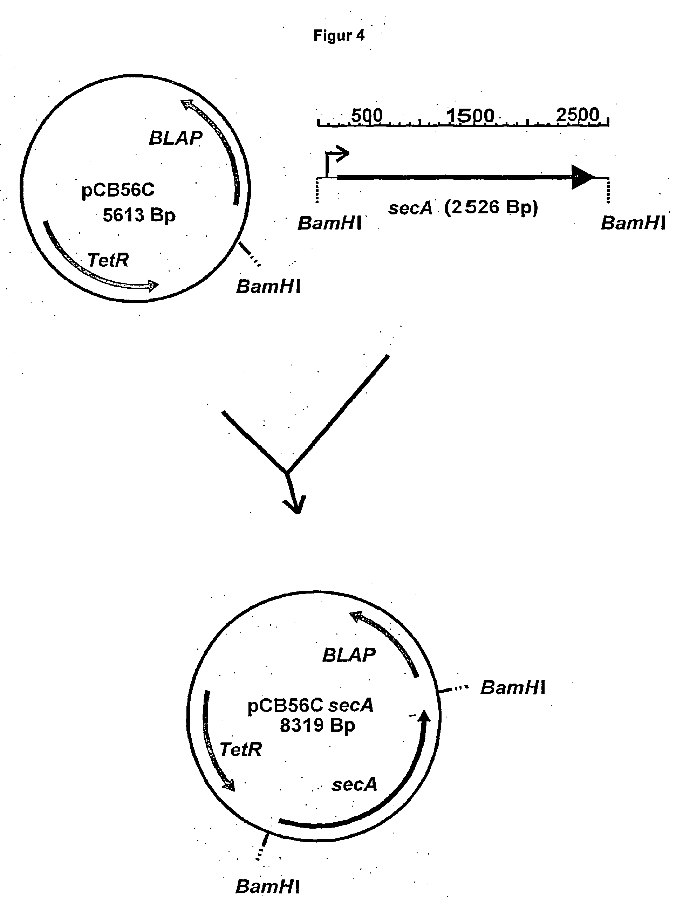

Das nach Beispiel 1 erhaltene *secA*-Gen wurde mit seinem eigenen Promotor mittels PCR ausgehend von chromosomaler DNA von *B. licheniformis* amplifiziert. Hierzu wurden, wie in Figur 4 dargestellt ist, anhand der DNA-Sequenz des Gens von *B. licheniformis* Primer ausgewählt, die am jeweiligen 5'-Ende eine *Bam*HI-Restriktionsschnittstelle besitzen. Über diese wurde das mit diesen Primern amplifizierte Fragment in die Schnittstelle des Plasmids pCB56C kloniert. Dieses ist in der Anmeldung WO 91/02792 A1 beschrieben und enthält das Gen für die Alkalische Protease aus *B. lentus* (*BLAP*).

Diese auch in Figur 4 dargestellte Klonierungsstrategie lieferte den 8319 bp großen Vektor pCB56C*secA*, der neben den Genen secA und *BLAP* auch eines enthält, das für eine Tetracyclin-Resistenz codiert.

Dieser Vektor pCB56C*secA* und zur Kontrolle der Ausgangsvektor pCB56C wurden in *B. licheniformis* transformiert, und zwar im Falle von pCB56C in den zur Bildung von SecA befähigten Wildtypstamm *B. licheniformis* (secA). Im Falle von pCB56C*secA* erfolgte die Transformation so, daß gleichzeitig das endogene *secA* inaktiviert wurde. Die Vorgehensweise dafür ist in Beispiel 3 beschrieben.

Auf diese Weise wurden die beiden Stämme *B. licheniformis* (Δ*secA)* pCB56C*secA* und *B. licheniformis (secA)* pCB56Cerhalten, die beide in der Lage waren, das plasmidal codierte Gen für die Alkalische Protease zu exprimieren. Sie werden in Beispiel 4 weiter untersucht.

### Beispiel 3

### Herstellung des Stammes B. licheniformis (ΔsecA) pCB56CsecA

Die Ausschaltung des Gens *secA* wurde mittels eines Deletionsvektors vorgenommen. Die Vorgehensweise folgt der Beschreibung von J.Vehmaanperä et al. (1991) in J. Biotechn., Band 19, Seiten 221-240.

Als Vektor zur *secA* Deletion wurde das in derselben Publikation beschriebene Plasmid pE194 ausgewählt. Der Vorteil dieses Deletionsvektors besteht darin, daß er einen Temperatur-abhängigen Replikationsursprung besitzt. Bei 33°C kann pE194 in der Zelle replizieren, so daß bei dieser Temperatur zunächst auf eine erfolgreiche Transformation selektiert werden kann. Anschließend werden die Zellen, die den Vektor enthalten, bei 42°C inkubiert. Bei dieser Temperatur repliziert der Deletionsvektor nicht mehr und es wird ein Selektionsdruck auf die Integration des Plasmids über einen der beiden homologen Bereiche (up- oder downstream Bereich von *secA*) in das Chromosom ausgeübt. Eine weitere homologe Rekombination über den anderen (zweiten) homologen Bereich führt dann zur *secA*-Deletion. Möglich wäre auch eine nochmalige Rekombination des ersten homologen Bereichs. Hierbei rekombiniert der Vektor wieder aus dem Chromosom heraus, so daß das chromosomale *secA* erhalten bliebe. Die *secA*-Deletion muß daher im Southern-Blot nach Restriktion der chromosomalen DNA mit geeigneten Enzymen oder mit Hilfe der PCR-Technik anhand der Größe des amplifizierten Bereichs nachgewiesen werden.

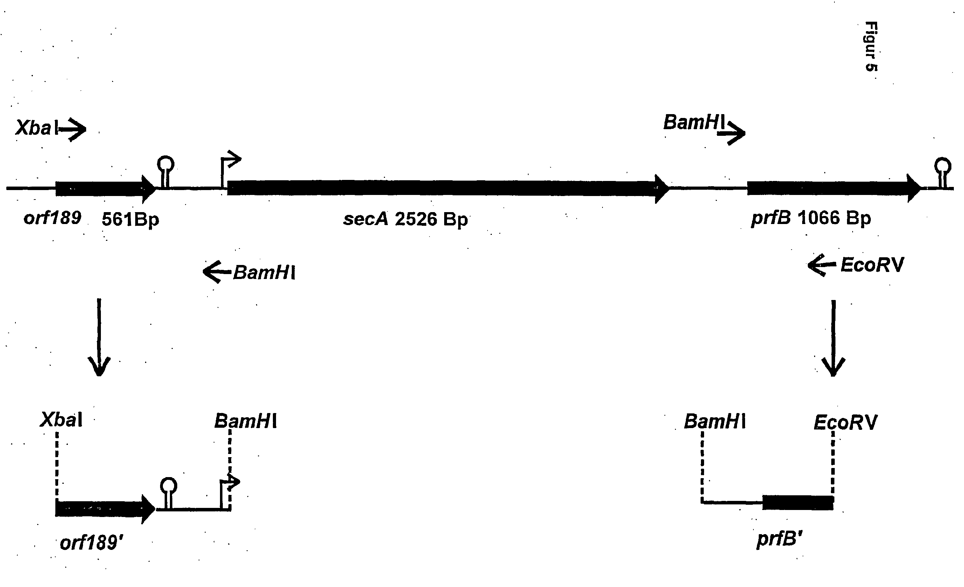

Zur Konstruktion des Deletionsvektors wurden die up- und downstream von *secA* gelegenen Bereiche (Figur 5) mittels PCR amplifiziert. Die Primer zur Amplifizierung und die mit diesen verbundenen Restriktionsschnittstellen zur anschließenden Klonierung (*Xba*I und *Eco*RV) wurden anhand der gemäß Beispiel 1 ermittelten DNA-Sequenz des *secA*/*prfB*-Locus von *B. licheniformis* ausgewählt. Bei der *secA*-Deletion ist zu beachten, daß das downstream von *secA* lokalisierte *prfB* mit *secA* in einem Operon liegt, also keinen eigenen Promotor besitzt (vergleiche Figur 2). Das *prfB* kodiert für das Protein RF2, welches im Zusammenhang mit der Proteinbiosynthese für das Ablösen des Proteins vom Ribosom sorgt. Um die Transkription des für die Proteinbiosynthese wichtigen *prfB* auch nach *secA*-Deletion zu gewährleisten, wurde der vor dem *secA* befindliche *orf189* mit eigenem Terminator und dem downstream lokalisierten *secA-*Promotor amplifiziert, so daß das *prfB* nach *secA*-Deletion direkt vom *secA*-Promotor aus transkribiert werden kann (Figur 5).

Die amplifizerten Bereiche (*orf189*' und *prfB*') wurden in einem Kontrollschritt in den *E. coli*-Vektor pBBRMCS2 zwischenkloniert. Die anschließende Sequenzierung des *orf989'prfB'*-Konstrukts zeigte, daß die amplifizierten Fragmente korrekt zusammenkloniert waren.

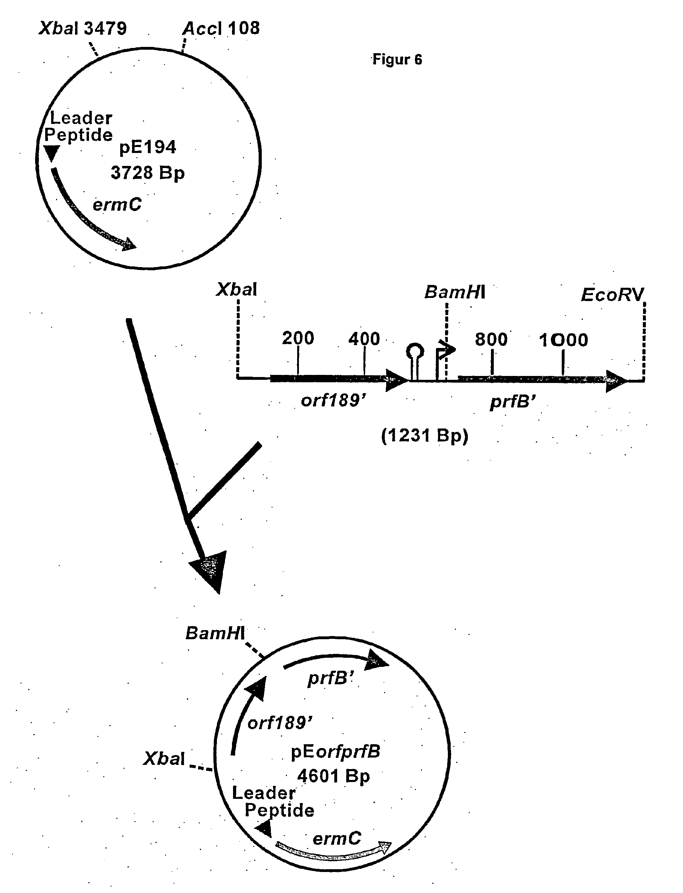

Das *orf189'prfB'*-Konstrukt wurde im nächsten Schritt in den zur Deletion ausgewählten Vektor pE 194 in *B. subtilis* DB104 umkloniert (Figur 6). Hierbei wurden mit der Methode der Protoplasten-Transformation nach Chang & Cohen, 1979, Transformanten erhalten, die den Deletionsvektor pEorfprfB trugen. Alle Arbeitsschritte wurden bei 33°C durchgeführt um eine Replikation des Vektors zu gewährleisten.

In einem nächsten Schritt wurde der in Beispiel 2 beschriebene Vektor pCB56C*secA* ebenfalls mittels der Methode der Protoplastentransformation in den das Plasmid pEorfprfB tragenden Wirtsstamm *B. licheniformis* transformiert. Die solcherart erhaltenen und mit üblichen Methoden als positiv identifizierten Transformanten wurden anschließend bei 42°C unter Selektionsdruck (Tetracyclin für pCB56C*secA* und Erythromycin für pEorfprfB) auf Anwesenheit beider Plasmide selektiert. Bei dieser Temperatur kann der Deletionsvektor nicht mehr replizieren und es überleben nur solche Zellen, bei denen der Vektor in das Chromosom integriert ist, wobei diese Integration mit höchster Wahrscheinlichkeit in homologen oder identischen Bereichen stattfindet. Durch Kultivierung bei 33°C ohne Erythromycin-Selektionsdruck kann dann nachfolgend die Excision des Deletionsvektors induziert werden, wobei das chromosomal codierte Gen *secA* vollständig aus dem Chromosom entfernt wird.

In der Zelle verbleibt das Plasmid pCB56C*secA*, welches die Fähigkeit zur Subtilisin-Synthese vermittelt sowie den essentiellen Translokations-Faktor SecA zur Verfügung stellt. Der auf diese Weise erhaltene Stamm wurde mit *B. licheniformis* (Δ*secA)* pCB56CsecA bezeichnet.

### Beispiel 4

### Untersuchung der Piasmidstabilität

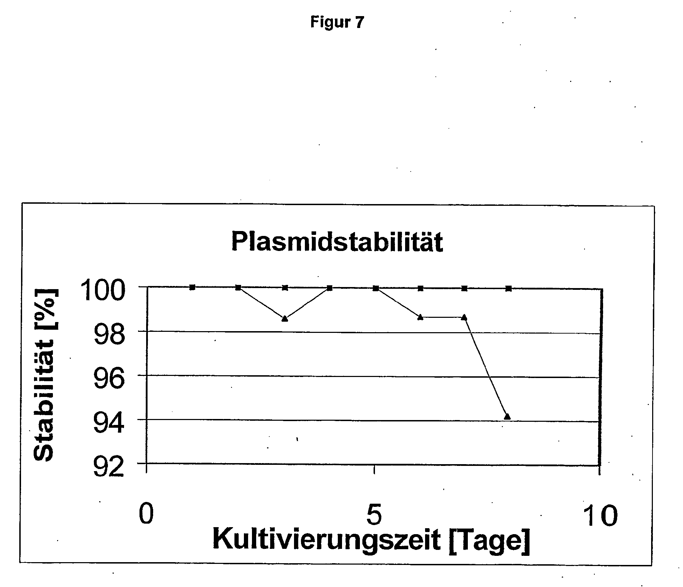

Zur Ermittlung der genetischen Stabilität des *secA*-tragenden Subtilisin-Plasmids pCB56CsecA wurden die nach den Beispielen 2 und 3 erhaltenen beiden Stämme *B. licheniformis* (*secA)* pCB56C und *B. licheniformis (*Δ*secA)* pCB56CsecA in einem Schüttelkolben-Experiment ohne Antibiotika-Zusatz in Flüssigmedium untersucht. Dazu wurden ausgehend von je einer einzelnen Kolonie eine Übernachtkultur angezogen und mit dieser jeweils 14 ml LB-Medium (nach Standardrezeptur) auf eine optische Dichte bei 600 nm (OD₆₀₀) von 0,05 beimpft. Die Kultivierung erfolgte in einem 100 ml-Erlenmeyer-Schüttelkolben. Nach jeweils 8 bis 16 Stunden wurden die Kulturen in 14 ml frisches Medium überimpft und hierbei wiederum eine OD₆₀₀ von 0,05 eingestellt. Die Kultivierung erfolgte über 8 Tage und Nächte; hierbei wurden die Kulturen insgesamt 16 mal überimpft. Jeden Tag wurden Verdünnungsreihen ausplattiert und eine zufällige Auswahl der erhaltenen Klone auf Protease-Test-Platten überführt. Das Ergebnis ist in Tabelle 2 und in Figur 7 dargestellt.

**Tabelle 2: Plasmidstabilität in den Transformanten B. licheniformis (secA) pCB56C (Kontrolle) und B. licheniformis (ΔsecA) pCB56CsecA, detektierbar anhand des jeweiligen Anteils der Klone mit Protease-Aktivität**

| | ***B. licheniformis (secA)* pCB56C** | | | ***B. licheniformis* (Δ*secA)* pCB56CsecA** | | |
|---|---|---|---|---|---|---|
| Zeit [Tage] | Zahl getesteter Klone | | Anteil der Klone mit Protease- | Zahl getesteter Klone | | Anteil der Klone mit Protease-Aktivität [%] |
| | gesamt | ohne Protease-Aktivität | Aktivität [%] | gesamt | ohne Protease-Aktivität | |
| 1 | 72 | 0 | 100 | 72 | 0 | 100 |
| 2 | 72 | 0 | 100 | 70 | 0 | 100 |
| 3 | 72 | 1 | 98,6 | 49 | 0 | 100 |
| 4 | 52 | 0 | 100 | 52 | 0 | 100 |
| 5 | 78 | 0 | 100 | 78 | 0 | 100 |
| 6 | 78 | 1 | 98,7 | 78 | 0 | 100 |
| 7 | 78 | 1 | 98,7 | 77 | 0 | 100 |
| 8 | 104 | 6 | 94,2 | 104 | 0 | 100 |

Zu jedem Kultivierungszeitpunkt zeigen alle getesteten Klone des Stammes *B. licheniformis* (Δ*secA)* pCB56C*secA* Protease-Aktvität, während beim Stamm *B. licheniformis (secA)* pCB56C einzelne Klone keine Protease-Aktivität mehr besitzen. Dies ist als Verlust des Plasmids pCB56C zu interpretieren; dieser Verlust wurde zusätzlich durch Plasmid-Minipräparation überprüft.

Anhand dieser Daten wird somit deutlich, daß bei Kultivierung in LB-Medium ohne Antibiotika-Zusatz, insbesonere ohne Tetrazyklin, für welches das Plasmid Resistenz verleihen würde, das *secA*-tragende Subtilisin-Plasmid pCB56C*secA* im Δ*secA*-Stamm stabil ist, während das Subtilisin-Plasmid pCB56C im Stamm ohne chromosomale *secA-*Deletion im Laufe der Kultivierung verloren geht. Das Gen *secA* aus *B. licheniformis* kann somit bei Übertragung auf einen Expressionsvektor die chromosomale SecA-Defizienz kurieren und auf diese Weise zur Selektion einer Bakterienkultur genutzt werden, die ein Gen für ein anderes Protein, in diesem Falle einer Alkalischen Protease exprimiert.

### Beschreibung der Figuren

**Figur 1****: Schematische Darstellung des Translations/Translokations-Apparats grampositiver Bakterien**
   Sinngemäß nach van Wely, K.H., Swaving, J., Freudl, R., Driessen, A.J. (2001); "Translocation of proteins across the cell envelope of Gram-positive bacteria", FEMS Microbiol Rev. 2001, 25(4), S. 437-54
**Figur 2****: Genort von *secA* in *B. subtilis***
   Man erkennt, daß in der secA-Region auch das Gen *prfB* liegt und eine zusammenhängende mRNA gebildet wird, so daß auch von einem *secA*/*prfB*-Operon gesprochen werden kann.
**Figur 3****: Restriktionskarte des Genorts *orf189* / *secA* / *prfB* in *B. licheniformis***
   Wie in Beispiel 1 dargestellt befinden sich in unmittelbarer Nachbarschaft zu *secA* das Gen *prfB* und ein *orf* auf einem ca. 5,5 kB großen Fragment, das durch Restriktion mit *Mun*I aus der genomischen DNA von *B. licheniformis* erhalten werden kann.
**Figur 4****: Herstellung eines Plasmids mit *secA*-Gen und Subtilisin-Gen**
   Wie in Beispiel 2 beschrieben wurde das *secA* über PCR amplifiziert und in einen Vector kloniert, der als Transgen die Alkalische Protease aus *B. lentus* enthielt.
**Figur 5****: Über PCR amplifizierte Bereiche von *secA* (up- und downstream)**
   Amplifizierung der up- und downstream-Bereiche von *secA* mit den zur Klonierung ausgewählten Restriktionsschnittstellen wie in Beispiel 3 beschrieben. Das 3' Ende von *orf189* wird mit eigenem Terminator und dem downstream liegenden secA-Promotor amplifiziert, so daß nach *secA*-Deletion das *prfB* direkt vom secA-Promotor aus transkribiert werden kann. Die abgeleiteten Abschnitte *orf189'* und *prfB'* umfassen jeweils 502 bp beziehungsweise 546 bp.
**Figur 6****: Konstruktion des Deletionsplasmids pEorfprfB**
   Die mittels PCR amplifizierten Bereiche wurden in *E.coli* zwischenkloniert , wiederum über *Xba*I und *EcoR*V ausgeschnitten und anschließend in die Restriktionsschnittstellen Xbal und *Acc*I in den Vektor pE194 ligiert.
**Figur 7****: Plasmidstabilität in den Transformanten *B. licheniformis (secA)* pCB56C (Kontrolle) und *B. licheniformis* (Δ*secA)* pCB56C*secA***
   Aufgetragen ist jeweils, wie in Beispiel 4 beschrieben, der Anteil der Klone mit Protease-Aktivität nach einer entsprechenden Anzahl von Tagen.
   - Quadrate:: *B. licheniformis* (Δ*secA)* pCB56C*secA*
   - Dreiecke:: *B. licheniformis (secA)* pCB56C (Kontrolle)

## Patentansprüche

1. Verfahren zur Selektion eines Mikroorganismus, **dadurch gekennzeichnet, daß**
(a) ein endogen vorhandenes für eine essentielle Translokations-Aktivität codierendes Gen inaktiviert und
(b) die nach (a) inaktivierte essentielle Translokations-Aktivität über einen Vektor kuriert wird.

2. Verfahren nach Anspruch 1, **dadurch gekennzeichnet, daß**
(c) der Vektor nach (b) ein Transgen trägt.

3. Verfahren nach Anspruch 1 oder 2, **dadurch gekennzeichnet, daß** es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

4. Verfahren nach Anspruch 3, **dadurch gekennzeichnet, daß** es sich bei der essentiellen Translokations-Aktivität um eine der folgenden Untereinheiten der Präprotein-Translokase handelt: SecA, SecY, SecE, SecD oder SecF, vorzugsweise um die Untereinheit SecA.

5. Verfahren nach einem der Ansprüche 1 bis 4, **dadurch gekennzeichnet, daß** das Kurieren nach (b) über eine zu der inaktivierten endogen vorhandenen essentiellen Translokations-Aktivität gleichwirkende Aktivität, vorzugsweise über eine genetisch verwandte, besonders bevorzugt über dieselbe Aktivität erfolgt.

6. Verfahren nach einem der Ansprüche 1 bis 5, **dadurch gekennzeichnet, daß** das Kurieren nach (b) über die die Translokationsaktivität wiederherstellenden Bereiche des Gens *secA* aus *Bacillus subtilis, Escherichia coli* oder *Bacillus licheniformis* erfolgt, die im Sequenzprotokoll unter SEQ ID NO. 1, SEQ ID NO. 3 beziehungsweise SEQ ID NO. 5 angegeben sind.

7. Verfahren nach einem der Ansprüche 2 bis 6, **dadurch gekennzeichnet, daß** die Inaktivierung nach (a) so erfolgt, daß eine Rekombination zwischen dem nach (a) inaktivierten Genbereich und dem homologen Bereich auf dem Vektor nach (b) verhindert wird, vorzugsweise unter vollständigem Verlust eines in dem betreffenden chromosomalen Gen enthaltenen Genabschnitts.

8. Verfahren nach einem der Ansprüche 1 bis 7, **dadurch gekennzeichnet, daß** die Inaktivierung nach (a) über einen Deletionsvektor erfolgt, vorzugsweise über einen Deletionsvektor mit einem extern regulierbaren Replikationsursprung, besonders bevorzugt über einen Deletionsvektor mit einem Temperatur-abhängigen Replikationsursprung.

9. Verfahren nach einem der Ansprüche 1 bis 8, **dadurch gekennzeichnet, daß** es sich bei dem Vektor nach (b) um ein in dem Mikroorganismus autonom replizierendes Plasmid handelt.

10. Verfahren nach Anspruch 9, **dadurch gekennzeichnet, daß** es sich bei dem Plasmid um eines sich in mehrfacher, vorzugsweise in vielfacher Kopienzahl etablierendes Plasmid handelt.

11. Verfahren nach einem der Ansprüche 1 bis 10, **dadurch gekennzeichnet, daß** es sich bei dem Mikroorganismus um einen gramnegativen Bakterien-Stamm handelt.

12. Verfahren nach Anspruch 11, **dadurch gekennzeichnet, daß** es sich um einen gramnegativen Bakterien-Stamm der Gattungen *E. coli* oder *Klebsiella* handelt, insbesondere um Derivate von *Escherichia coli* K12, von *Escherichia coli* B oder *Klebsiella planticola,* und ganz besonders um Derivate der Stämme *Escherichia coli* BL21 (DE3), *E. coli* RV308, *E. coli* DH5α, *E.coli* JM109, *E. coli* XL-1 oder *Klebsiella planticola* (Rf).

13. Verfahren nach einem der Ansprüche 1 bis 10, **dadurch gekennzeichnet, daß** es sich bei dem Mikroorganismus um einen grampositiven Bakterien-Stamm handelt.

14. Verfahren nach Anspruch 13, **dadurch gekennzeichnet, daß** es sich um einen grampositiven Bakterien-Stamm der Gattungen *Staphylococcus, Corynebakterien* oder *Bacillus* handelt, insbesondere der Spezies *Staphylococcus carnosus*, *Corynebacterium glutamicum, Bacillus subtilis, B. licheniformis, B. amyloliquefaciens, B. globigii* oder *B. lentus,* und ganz besonders um Derivate der Stämme *B. licheniformis* oder *B. amylotiquefaciens*.

15. Verfahren nach einem der Ansprüche 2 bis 14, **dadurch gekennzeichnet, daß** es sich bei dem Transgen nach (c) um eines handelt, das für ein Nicht-Enzym-Protein codiert, insbesondere für ein pharmakologisch relevantes Protein, ganz besonders für Insulin oder Calcitonin.

16. Verfahren nach einem der Ansprüche 2 bis 14, **dadurch gekennzeichnet, daß** es sich bei dem Transgen nach (c) um eines handelt, das für ein Enzym codiert, vorzugsweise für ein hydrolytisches Enzym oder eine Oxidoreduktase, besonders bevorzugt für eine Protease, Amylase, Hemicellulase, Cellulase, Lipase, Cutinase, Oxidase, Peroxidase oder Laccase.

17. Verfahren zur Herstellung eines Proteins durch Kultivierung von Zellen eines Mikroorganismus-Stammes, **gekennzeichnet durch** ein Selektionsverfahren nach einem der Ansprüche 1 bits 16.

18. Verfahren nach Anspruch 17, **gekennzeichnet durch** ein Selektionsverfahren nach einem der Ansprüche 2 bis 16, wobei das Transgen nach (c) für das über dieses Verfahren hergestellte Protein codiert.

19. Verfahren nach einem der Ansprüche 17 bis 18, **dadurch gekennzeichnet, daß** die Kultivierung der Mikroorganismen in einem flüssigen Medium erfolgt, vorzugsweise in einem Fermenter.

20. Verfahren nach einem der Ansprüche 17 bis 19, **dadurch gekennzeichnet, daß** das interessierende Protein ins umgebende Medium sekretiert wird.

21. Mikroorganismus, **erhalten durch** ein Selektionsverfahren nach einem der Ansprüche 1 bis 16.

22. Mikroorganismus nach Anspruch 21, **dadurch gekennzeichnet, daß** das Transgen exprimiert wird.

23. Mikroorganismus nach Anspruch 22, **dadurch gekennzeichnet, daß** das Transgen sekretiert wird.

24. Verwendung eines für eine essentielle Translokations-Aktivität codierenden Gens zur Selektion eines Mikroorganismus, **dadurch gekennzeichnet, daß**
(a) diese in dem Mikroorganismus endogen vorhandene essentielle Translokations-Aktivität inaktiviert wird und
(b) die nach (a) inaktivierte essentielle Translokations-Aktivität über einen Vektor kuriert wird.

25. Verwendung nach Anspruch 24, **dadurch gekennzeichnet, daß**
(c) der Vektor nach (b) ein Transgen trägt.

26. Verwendung nach Anspruch 24 oder 25, **dadurch gekennzeichnet, daß** es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

27. Verwendung nach Anspruch 26, **dadurch gekennzeichnet, daß** es sich bei der essentiellen Translokations-Aktivität um eine der folgenden Untereinheiten der Präprotein-Translokase handelt: SecA, SecY, SecE, SecD oder SecF, vorzugsweise um die Untereinheit SecA.

28. Verwendung nach einem der Ansprüche 24 bis 27, **dadurch gekennzeichnet, daß** das Kurieren nach (b) über eine zu der inaktivierten endogen vorhandenen essentiellen Translokations-Aktivität gleichwirkende Aktivität, vorzugsweise über eine genetisch verwandte, besonders bevorzugt über dieselbe Aktivität erfolgt.

29. Verwendung nach einem der Ansprüche 24 bis 28, **dadurch gekennzeichnet, daß** das Kurieren nach (b) über die die Translokationsaktivität wiederherstellenden Bereiche des Gens *secA* aus *Bacillus subtilis, Escherichia coli* oder *Bacillus licheniformis* erfolgt, die im Sequenzprotokoll unter SEQ ID NO. 1, SEQ ID NO. 3 beziehungsweise SEQ ID NO. 5 angegeben sind.

30. Verwendung nach einem der Ansprüche 24 bis 29, **dadurch gekennzeichnet, daß** es sich bei dem Vektor nach (b) um ein in dem Mikroorganismus autonom replizierendes Plasmid handelt.

31. Verwendung nach Anspruch 30, **dadurch gekennzeichnet, daß** es sich bei dem Plasmid um eines sich in mehrfacher, vorzugsweise in vielfacher Kopienzahl etablierendes Plasmid handelt.

32. Vektor, der ein Gen für eine essentielle Translokations-Aktivität und ein Transgen trägt, welches, wenn es als einziges Transgen vorhanden ist, nicht für eine Antibiotikum-Resistenz codiert.

33. Vektor nach Anspruch 32, **dadurch gekennzeichnet, daß** das Transgen für ein pharmakologisch relevantes Nicht-Enzym-Protein oder für ein hydrolytisches Enzym oder für eine Oxidoreduktase codiert.

34. Vektor nach Anspruch 32 oder 33, **dadurch gekennzeichnet, daß** die von ihm codierte Translokations-Aktivität eine in einem Mikroorganismenstamm endogen vorhandene, inaktivierte essentielle Translokations-Aktivität zu kurieren vermag, vorzugsweise über eine genetisch verwandte, besonders bevorzugt über dieselbe Aktivität.

35. Vektor nach einem der Ansprüche 32 bis 34, **dadurch gekennzeichnet, daß** es sich bei der essentiellen Translokations-Aktivität um einen der folgenden Faktoren handelt: SecA, SecY, SecE, SecD, SecF, Signalpeptidase, b-SRP (Ffh oder Ffs / Scr), FtsY / Srb, PrsA oder YajC.

36. Vektor nach Anspruch 35, **dadurch gekennzeichnet, daß** es sich bei der essentiellen Translokations-Aktivität um eine der folgenden Untereinheiten der Präprotein-Translokase handelt: SecA, SecY, SecE, SecD oder SecF, vorzugsweise um die Untereinheit SecA.

37. Vektor nach einem der Ansprüche 32 bis 36, **dadurch gekennzeichnet, daß** es sich bei der essentiellen Translokations-Aktivität um eines der Gene *secA* aus *Bacillus subtilis, Escherichia coli* oder *Bacillus licheniformis* handelt, die im Sequenzprotokoll unter SEQ ID NO. 1, SEQ ID NO. 3 beziehungsweise SEQ ID NO. 5 angegeben sind.

38. Vektor nach einem der Ansprüche 32 bis 37, **dadurch gekennzeichnet, daß** es sich um ein in dem Mikroorganismus autonom replizierendes Plasmid handelt.

39. Vektor nach Anspruch 38, **dadurch gekennzeichnet, daß** es sich bei dem Plasmid um eines sich in mehrfacher, vorzugsweise in vielfacher Kopienzahl etablierendes Plasmid handelt.

## Claims

1. A process for the selection of a microorganism, **characterized in that**
(a) an endogenously present gene coding for an essential translocation activity is inactivated and
(b) the essential translocation activity inactivated according to (a) is cured by means of a vector.

2. The process as claimed in claim 1, **characterized in that**
(c) the vector according to (b) carries a transgene.

3. The process as claimed in claim 1 or 2, **characterized in that** the essential translocation activity is one of the following factors: SecA, SecY, SecE, SecD, SecF, signal peptidase, b-SRP (Ffh or Ffs/Scr), FtsY/Srb, PrsA or YajC.

4. The process as claimed in claim 3, **characterized in that** the essential translocation activity is one of the following subunits of the preprotein translocase: SecA, SecY, SecE, SecD or SecF, preferably the subunit SecA.

5. The process as claimed in one of claims 1 to 4, **characterized in that** the curing according to (b) takes place by means of an activity acting identically to the inactivated endogenously present essential translocation activity, preferably by means of a genetically related activity, particularly preferably by means of the same activity.

6. The process as claimed in one of claims 1 to 5, **characterized in that** the curing according to (b) takes place by means of the regions of the gene *secA* from *Bacillus subtilis, Escherichia coli* and *Bacillus licheniformis* restoring the translocation activity, which are indicated in the sequence listing under SEQ ID NO. 1, SEQ ID NO. 3 and SEQ ID NO. 5 respectively.

7. The process as claimed in one of claims 2 to 6, **characterized in that** the inactivation according to (a) is carried out such that a recombination between the gene region inactivated according to (a) and the homologous region on the vector according to (b) is prevented, preferably with complete loss of a gene section contained in the chromosomal gene concerned.

8. The process as claimed in one of claims 1 to 7, **characterized in that** the inactivation according to (a) takes place by means of a deletion vector, preferably by means of a deletion vector having an externally regulatable replication origin, particularly preferably by means of a deletion vector having a temperature-dependent replication origin.

9. The process as claimed in one of claims 1 to 8, **characterized in that** the vector according to (b) is a plasmid replicating autonomously in the microorganism.

10. The process as claimed in claim 9, **characterized in that** the plasmid is a plasmid establishing in a plural, preferably in a multiple, copy number.

11. The process as claimed in one of claims 1 to 10, **characterized in that** the microorganism is a gram-negative strain of bacteria.

12. The process as claimed in claim 11, **characterized in that** a gram-negative strain of bacteria of the genera *E. coli* or *Klebsiella* is concerned, in particular derivatives of *Escherichia coli* K12, of *Escherichia coli* B or *Klebsiella planticola,* and very particularly derivatives of the strains *Escherichia coli* BL21 (DE3), *E. coli* RV308, *E. coli* DH5α, *E. coli* JM109, *E. coli* XL-1 or *Klebsiella planticola* (Rf).

13. The process as claimed in one of claims 1 to 10, **characterized in that** the microorganism is a gram-positive strain of bacteria.

14. The process as claimed in claim 13, **characterized in that** it is a gram-positive strain of bacteria of the genera *Staphylococcus, Corynebacteria* or *Bacillus,* in particular of the species *Staphylococcus carnosus, Corynebacterium glutamicum, Bacillus subtilis, B. licheniformis, B. amyloliquefaciens, B. globigii* or *B. lentus,* and very particularly derivatives of the strains *B. licheniformis* or *B. amyloliquefaciens.*

15. The process as claimed in one of claims 2 to 14, **characterized in that** the transgene according to (c) is one which codes for a nonenzyme protein, in particular for a pharmacologically relevant protein, very particularly for insulin or calcitonin.

16. The process as claimed in one of claims 2 to 14, **characterized in that** the transgene according to (c) is one which codes for an enzyme, preferably for a hydrolytic enzyme or an oxidoreductase, particularly preferably for a protease, amylase, hemicellulase, cellulase, lipase, cutinase, oxidase, peroxidase or laccase.

17. A process for the preparation of a protein by culturing cells of a microorganism strain, **characterized by** a selection process as claimed in one of claims 1 to 16.

18. The process as claimed in claim 17, **characterized by** a selection process as claimed in one of claims 2 to 16, the transgene according to (c) coding for the protein prepared by means of this process.

19. The process as claimed in one of claims 17 to 18, **characterized in that** the culturing of the microorganisms takes place in a liquid medium, preferably in a fermenter.

20. The process as claimed in one of claims 17 to 19, **characterized in that** the protein of interest is secreted into the surrounding medium.

21. A microorganism, **obtained by** a selection process as claimed in one of claims 1 to 16.

22. The microorganism as claimed in claim 21, **characterized in that** the transgene is expressed.

23. The microorganism as claimed in claim 22, **characterized in that** the transgene is secreted.

24. The use of a gene coding for an essential translocation activity for the selection of a microorganism, **characterized in that**
(a) this essential translocation activity endogenously present in the microorganism is inactivated and
(b) the essential translocation activity inactivated according to (a) is cured by means of a vector.

25. The use as claimed in claim 24, **characterized in that**
(c) the vector according to (b) carries a transgene.

26. The use as claimed in claim 24 or 25, **characterized in that** the essential translocation activity is one of the following factors: SecA, SecY, SecE, SecD, SecF, signal peptidase, b-SRP (Ffh or Ffs/Scr), FtsY/Srb, PrsA or YajC.

27. The use as claimed in claim 26, **characterized in that** the essential translocation activity is one of the following subunits of the preprotein translocase: SecA, SecY, SecE, SecD or SecF, preferably the subunit SecA.

28. The use as claimed in one of claims 24 to 27, **characterized in that** the curing according to (b) takes place by means of an activity acting identically to the inactivated endogenously present essential translocation activity, preferably by means of a genetically related activity, particularly preferably by means of the same activity.

29. The use as claimed in one of claims 24 to 28, **characterized in that** the curing according to (b) takes place by means of the regions of the gene *secA* from *Bacillus subtilis, Escherichia coli* and *Bacillus licheniformis* restoring the translocation activity, which are indicated in the sequence listing under SEQ ID NO. 1, SEQ ID NO. 3 and SEQ ID NO. 5 respectively.

30. The use as claimed in one of claims 24 to 29, **characterized in that** the vector according to (b) is a plasmid replicating autonomously in the microorganism.

31. The use as claimed in claim 30, **characterized in that** the plasmid is a plasmid establishing in a plural, preferably in a multiple, copy number.

32. A vector which carries a gene for an essential translocation activity and a transgene which, if it is present as the only transgene, does not code for an antibiotic resistance.

33. The vector as claimed in claim 32, **characterized in that** the transgene codes for a pharmacologically relevant nonenzyme protein or for a hydrolytic enzyme or for an oxidoreductase.

34. The vector as claimed in claim 32 or 33, **characterized in that** the translocation activity encoded by it is able to cure an inactivated essential translocation activity endogenously present in a microorganism strain, preferably by means of a genetically related activity, particularly preferably by means of the same activity.

35. The vector as claimed in one of claims 32 to 34, **characterized in that** the essential translocation activity is one of the following factors: SecA, SecY, SecE, SecD, SecF, signal peptidase, b-SRP (Ffh or Ffs/Scr), FtsY/Srb, PrsA or YajC.

36. The vector as claimed in claim 35, **characterized in that** the essential translocation activity is one of the following subunits of the preprotein translocase: SecA, SecY, SecE, SecD or SecF, preferably the subunit SecA.

37. The vector as claimed in one of claims 32 to 36, **characterized in that** the essential translocation activity is one of the genes *secA* from *Bacillus subtilis, Escherichia* coli or *Bacillus licheniformis,* which are indicated in the sequence listing under SEQ ID NO. 1, SEQ ID NO. 3 and SEQ ID NO. 5 respectively.

38. The vector as claimed in one of claims 32 to 37, **characterized in that** a plasmid replicating autonomously in the microorganism is concerned.

39. The vector as claimed in claim 38, **characterized in that** the plasmid is one establishing in a plural, preferably in a multiple, copy number.

## Revendications

1. Procédé pour la sélection d'un microorganisme, **caractérisé**
(a) **en ce qu'**un gène présent de manière endogène, codant pour une activité de translocation essentielle est inactivé et
(b) **en ce que** l'activité de translocation essentielle inactivée selon (a) est guérie via un vecteur.

2. Procédé selon la revendication 1, **caractérisé**
(c) en ce que le vecteur selon (b) porte un transgène.

3. Procédé selon la revendication 1 ou 2, **caractérisé en ce qu'**il s'agit, pour l'activité de translocation essentielle, d'un des facteurs suivants : SecA, SecY, SecE, SecD, SecF, signalpeptidase, b-SRP (Ffh ou Ffs/Scr), FtsY/Srb, PrsA ou YajC.

4. Procédé selon la revendication 3, **caractérisé en ce qu'**il s'agit, pour l'activité de translocation essentielle, d'une des sous-unités suivantes de la translocase de préprotéine : SecA, SecY, SecE, SecD ou SecF, de préférence de la sous-unité SecA.

5. Procédé selon l'une quelconque des revendications 1 à 4, **caractérisé en ce que** la guérison selon (b) se produit via une activité agissant de la même manière que l'activité de translocation essentielle présente de manière endogène inactivée, de préférence via une activité génétiquement apparentée, de manière particulièrement préférée via la même activité.

6. Procédé selon l'une quelconque des revendications 1 à 5, **caractérisé en ce que** la guérison selon (b) se produit via des domaines réparant l'activité de translocation du gène secA de Bacillus subtilis, d'Escherichia coli ou de Bacillus licheniformis, qui sont indiqués dans le protocole de séquences sous la SEQ ID NO. 1, la SEQ ID NO. 3 ou, selon le cas, la SEQ ID NO. 5.

7. Procédé selon l'une quelconque des revendications 2 à 6, **caractérisé en ce que** l'inactivation selon (a) est réalisée de manière telle qu'une recombinaison entre le domaine génique inactivé selon (a) et le domaine homologue sur le vecteur selon (b) est empêchée, de préférence avec perte complète d'une section génique contenue dans le gène chromosomique concerné.

8. Procédé selon l'une quelconque des revendications 1 à 7, **caractérisé en ce que** l'inactivation selon (a) se produit via un vecteur de délétion, de préférence via un vecteur de délétion avec une origine de réplication régulable de manière externe, de manière particulièrement préférée via un vecteur de délétion avec une origine de réplication dépendant de la température.

9. Procédé selon l'une quelconque des revendications 1 à 8, **caractérisé en ce qu'**il s'agit, pour le vecteur (b) d'un plasmide à réplication autonome dans le microorganisme.

10. Procédé selon la revendication 9, **caractérisé en ce qu'**il s'agit pour le plasmide d'un plasmide s'établissant dans plusieurs copies, de préférence dans un grand nombre de copies.

11. Procédé selon l'une quelconque des revendications 1 à 10, **caractérisé en ce qu'**il s'agit pour le microorganisme d'une souche de bactéries gram-négatives.

12. Procédé selon la revendication 11, **caractérisé en ce qu'**il s'agit d'une souche de bactéries gram-négatives des genres E. coli ou Klebsiella, en particulier de dérivés d'Escherichia coli K12, d'Escherichia coli B ou de Klebsiella planticola, et tout particulièrement de dérivés des souches Escherichia coli BL21 (DE3), E. coli RV308, E. coli DH5α, E. coli JM109, E. coli XL-1 ou Klebsiella planticola (Rf).

13. Procédé selon l'une quelconque des revendications 1 à 10, **caractérisé en ce qu'**il s'agit pour le microorganisme d'une souche de bactéries gram-positives.

14. Procédé selon la revendication 13, **caractérisé en ce qu'**il s'agit d'une souche de bactéries gram-positives des genres Staphylococcus, Corynebacterium ou Bacillus, de préférence des espèces Staphylococcus carnosus, Corynebacterium glutamicum, Bacillus subtilis, B. licheniformis, B. amyloliquefaciens, B. globigii ou B. lentus, et tout particulièrement de dérivés des souches B. licheniformis ou B. amyloliquefaciens.

15. Procédé selon l'une quelconque des revendications 2 à 14, **caractérisé en ce qu'**il s'agit, pour le transgène selon (c), d'un transgène qui code pour une protéine non enzyme, en particulier pour une protéine présentant un intérêt pharmacologique, tout particulièrement pour l'insuline ou la calcitonine.

16. Procédé selon l'une quelconque des revendications 2 à 14, **caractérisé en ce qu'**il s'agit pour le transgène selon (c) d'un transgène qui code pour une enzyme, de préférence pour une enzyme hydrolytique ou une oxydoréductase, de manière particulièrement préférée pour une protéase, une amylase, une hémicellulase, une cellulase, une lipase, une cutinase, une oxydase, une peroxydase ou une laccase.

17. Procédé pour la production d'une protéine par culture de cellules d'une souche de microorganismes, **caractérisé par** un procédé de sélection selon l'une quelconque des revendications 1 à 16.

18. Procédé selon la revendication 17, **caractérisé par** un procédé de sélection selon l'une quelconque des revendications 2 à 16, où le transgène selon (c) code pour la protéine produite selon ce procédé.

19. Procédé selon l'une quelconque des revendications 17 à 18, **caractérisé en ce que** la culture des microorganismes est réalisée dans un milieu liquide, de préférence dans un fermenteur.

20. Procédé selon l'une quelconque des revendications 17 à 19, **caractérisé en ce que** la protéine à laquelle on s'intéresse est sécrétée dans le milieu environnant.

21. Microorganisme obtenu par un procédé de sélection selon l'une quelconque des revendications 1 à 16.

22. Microorganisme selon la revendication 21, **caractérisé en ce que** le transgène est exprimé.

23. Microorganisme selon la revendication 22, **caractérisé en ce que** le transgène est sécrété.

24. Utilisation d'un gène codant pour une activité de translocation essentielle pour la sélection d'un microorganisme, **caractérisée en ce**
(a) **que** cette activité de translocation essentielle présente de manière endogène dans le microorganisme est inactivée et
(b) **que** l'activité de translocation essentielle inactivée selon (a) est guérie via un vecteur.

25. Utilisation selon la revendication 24, **caractérisée en ce que** (c) le vecteur selon (b) porte un transgène.

26. Utilisation selon la revendication 24 ou 25, **caractérisée en ce qu'**il s'agit, pour l'activité de translocation essentielle, d'un des facteurs suivants : SecA, SecY, SecE, SecD, SecF, signalpeptidase, b-SRP (Ffh ou Ffs/Scr), FtsY/Srb, PrsA ou YajC.

27. Utilisation selon la revendication 26, **caractérisée en ce qu'**il s'agit, pour l'activité de translocation essentielle, d'une des sous-unités suivantes de la translocase de préprotéine : SecA, SecY, SecE, SecD ou SecF, de préférence de la sous-unité SecA.

28. Utilisation selon l'une quelconque des revendications 24 à 27, **caractérisée en ce que** la guérison selon (b) se produit via une activité agissant de la même manière que l'activité de translocation essentielle présente de manière endogène inactivée, de préférence via une activité génétiquement apparentée, de manière particulièrement préférée via la même activité.

29. Utilisation selon l'une quelconque des revendications 24 à 28, **caractérisée en ce que** la guérison selon (b) se produit via des domaines réparant l'activité de translocation du gène secA de Bacillus subtilis, d'Escherichia coli ou de Bacillus licheniformis, qui sont indiqués dans le protocole de séquences sous la SEQ ID NO. 1, la SEQ ID NO. 3 ou, selon le cas, la SEQ ID NO. 5.

30. Utilisation selon l'une quelconque des revendications 24 à 29, **caractérisée en ce qu'**il s'agit, pour le vecteur (b) d'un plasmide à réplication autonome dans le microorganisme.

31. Utilisation selon la revendication 30, **caractérisée en ce qu'**il s'agit pour le plasmide d'un plasmide s'établissant dans plusieurs copies, de préférence dans un grand nombre de copies.

32. Vecteur, qui porte un gène pour une activité de translocation essentielle et un transgène, qui, lorsqu'il est présent comme transgène unique, ne code pas pour une résistance à un antibiotique.

33. Vecteur selon la revendication 32, **caractérisé en ce que** le transgène code pour une protéine non enzyme présentant un intérêt pharmacologique ou pour une enzyme hydrolytique ou pour une oxydoréductase.

34. Vecteur selon la revendication 32 ou 33, **caractérisé en ce que** l'activité de translocation pour laquelle il code permet de guérir une activité de translocation essentielle inactivée, présente de manière endogène dans une souche de microorganismes, de préférence via une activité génétiquement apparentée, de manière particulièrement préférée via la même activité.

35. Vecteur selon l'une quelconque des revendications 32 à 34, **caractérisé en ce qu'**il s'agit, pour l'activité de translocation essentielle, d'un des facteurs suivants : SecA, SecY, SecE, SecD, SecF, signalpeptidase, b-SRP (Ffh ou Ffs/Scr), FtsY/Srb, PrsA ou YajC.

36. Vecteur selon la revendication 35, **caractérisé en ce qu'**il s'agit, pour l'activité de translocation essentielle, d'une des sous-unités suivantes de la translocase de préprotéine : SecA, SecY, SecE, SecD ou SecF, de préférence de la sous-unité SecA.

37. Vecteur selon l'une quelconque des revendications 32 à 36, **caractérisé en ce qu'**il s'agit, pour l'activité de translocation essentielle d'une activité des gènes secA de Bacillus subtilis, d'Escherichia coli ou de Bacillus licheniformis, qui sont indiqués dans le protocole de séquences sous la SEQ ID NO. 1, la SEQ ID NO. 3 ou, selon le cas, la SEQ ID NO. 5.

38. Vecteur selon l'une quelconque des revendications 32 à 37, **caractérisé en ce qu'**il s'agit d'un plasmide à réplication autonome dans le microorganisme.

39. Vecteur selon la revendication 38, **caractérisé en ce qu'**il s'agit pour le plasmide d'un plasmide s'établissant dans plusieurs copies, de préférence dans un grand nombre de copies.


### SEQUENZPROTOKOLL

<110> Henkel Kommanditgesellschaft auf Aktien
<120> Ein Translokationsenzym als Selektionsmarker
<130> H 05599 PCT
<140>
   <141>
<150> DE10309557.8
   <151> 2003-03-04
<160> 6
<170> PatentIn Ver. 2.1
<210> 1
   <211> 4148
   <212> DNA
   <213> Bacillus subtilis
<220>
   <221> CDS
   <222> (544)..(3069)
<400> 1
   gtccgaggtg cataacgagg atatgtacaa cgcaattgat ctcgcaacaa acaaactgga 60
   acgtcaaatc cgtaagcata aaacgaaagt aaaccgtaaa ttccgtgagc agggctctcc 120
   aaaatattta ttggcaaacg gtcttggctc tgatacagat attgcggttc aggatgacat 180
   agaagaggag gagagcttgg acatcgtccg tcagaaacgc tttaatttaa agccgatgga 240
   tagtgaagaa gcgatcttgc aaatgaatat gctcggccat aatttctttg ttttcacaaa 300
   tgcggaaaca aaccttacaa atgtcgtgta ccgcagaaat gacgggaaat atggcttaat 360
   tgaaccgact gaataatgaa gagaagcctt ccgtgatgtc cgcggaaggt tttgtttttt 420
   cttatttgca aattctttgg aaataacaaa aggtatgata tgataatgag aggtatacat 480
   ggactagtaa attatttata catgcctcta aaataggcgt gtgatgatag aggagcgtta 540
   taa atg ctt gga att tta aat aaa atg ttt gat cca aca aaa cgt acg    588
       Met Leu Gly Ile Leu Asn Lys Met Phe Asp Pro Thr Lys Arg Thr
        1               5                  10                  15
   ctg aat aga tac gaa aaa att gct aac gat att gat gcg att cgc gga    636
   Leu Asn Arg Tyr Glu Lys Ile Ala Asn Asp Ile Asp Ala Ile Arg Gly
                    20                  25                  30
   gac tat gaa aat ctc tct gac gac gca ttg aaa cat aaa aca att gaa    684
   Asp Tyr Glu Asn Leu Ser Asp Asp Ala Leu Lys His Lys Thr Ile Glu
                35                  40                  45
   ttt aaa gag cgt ctt gaa aaa ggg gcg aca acg gat gat ctt ctt gtt    732
   Phe Lys Glu Arg Leu Glu Lys Gly Ala Thr Thr Asp Asp Leu Leu Val
           50                  55                  60
   gaa gct ttc gct gtt gtt cga gaa gct tca cgc cgc gta aca ggc atg    780
   Glu Ala Phe Ala Val Val Arg Glu Ala Ser Arg Arg Val Thr Gly Met
       65                  70                  75
   ttt ccg ttt aaa gtc cag ctc atg ggg ggc gtg gcg ctt cat gac gga    828
   Phe Pro Phe Lys Val Gln Leu Met Gly Gly Val Ala Leu His Asp Gly
   80                  85                  90                  95
   aat ata gcg gaa atg aaa aca ggg gaa ggg aaa aca tta acg tct acc    876
   Asn Ile Ala Glu Met Lys Thr Gly Glu Gly Lys Thr Leu Thr Ser Thr
                   100                 105                 110
   ctg cct gtt tat tta aat gcg tta acc ggt aaa ggc gta cac gtc gtg    924
   Leu Pro Val Tyr Leu Asn Ala Leu Thr Gly Lys Gly Val His Val Val
               115                 120                 125
   act gtc aac gaa tac ttg gca agc cgt gac gct gag caa atg ggg aaa    972
   Thr Val Asn Glu Tyr Leu Ala Ser Arg Asp Ala Glu Gln Met Gly Lys
           130                 135                 140
   att ttc gag ttt ctc ggt ttg act gtc ggt ttg aat tta aac tca atg    1020
   Ile Phe Glu Phe Leu Gly Leu Thr Val Gly Leu Asn Leu Asn Ser Met
       145                 150                 155
   tca aaa gac gaa aaa cgg gaa gct tat gcc gct gat att act tac tcc    1068
   Ser Lys Asp Glu Lys Arg Glu Ala Tyr Ala Ala Asp Ile Thr Tyr Ser
   160                 165                 170                 175
   aca aac aac gag ctt ggc ttc gac tat ttg cgt gac aat atg gtt ctt    1116
   Thr Asn Asn Glu Leu Gly Phe Asp Tyr Leu Arg Asp Asn Met Val Leu
                   180                 185                 190
   tat aaa gag cag atg gtt cag cgc ccg ctt cat ttt gcg gta ata gat    1164
   Tyr Lys Glu Gln Met Val Gln Arg Pro Leu His Phe Ala Val Ile Asp
               195                 200                 205
   gaa gtt gac tct att tta att gat gaa gca aga aca ccg ctt atc att    1212
   Glu Val Asp Ser Ile Leu Ile Asp Glu Ala Arg Thr Pro Leu Ile Ile
           210                 215                 220
   tct gga caa gct gca aaa tcc act aag ctg tac gta cag gca aat gct    1260
   Ser Gly Gln Ala Ala Lys Ser Thr Lys Leu Tyr Val Gln Ala Asn Ala
       225                 230                 235
   ttt gtc cgc acg tta aaa gcg gag aag gat tac acg tac gat atc aaa    1308
   Phe Val Arg Thr Leu Lys Ala Glu Lys Asp Tyr Thr Tyr Asp Ile Lys
   240                 245                 250                 255
   aca aaa gct gta cag ctt act gaa gaa gga atg acg aag gcg gaa aaa    1356
   Thr Lys Ala Val Gln Leu Thr Glu Glu Gly Met Thr Lys Ala Glu Lys
                   260                 265                 270
   gca ttc ggc atc gat aac ctc ttt gat gtg aag cat gtc gcg ctc aac    1404
   Ala Phe Gly Ile Asp Asn Leu Phe Asp Val Lys His Val Ala Leu Asn
               275                 280                 285
   cac cat atc aac cag gcc tta aaa gct cac gtt gcg atg caa aag gac     1452
   His His Ile Asn Gln Ala Leu Lys Ala His Val Ala Met Gln Lys Asp
           290                 295                 300
   gtt gac tat gta gtg gaa gac gga cag gtt gtt att gtt gat tcc ttc     1500
   Val Asp Tyr Val Val Glu Asp Gly Gln Val Val Ile Val Asp Ser Phe
       305                 310                 315
   acg gga cgt ctg atg aaa ggc cgc cgc tac agt gag ggg ctt cac caa     1548
   Thr Gly Arg Leu Met Lys Gly Arg Arg Tyr Ser Glu Gly Leu His Gln
   320                 325                 330                 335
   gcg att gaa gca aag gaa ggg ctt gag att caa aac gaa agc atg acc     1596
   Ala Ile Glu Ala Lys Glu Gly Leu Glu Ile Gln Asn Glu Ser Met Thr
                   340                 345                 350
   ttg gcg acg att acg ttc caa aac tac ttc cga atg tac gaa aaa ctt     1644
   Leu Ala Thr Ile Thr Phe Gln Asn Tyr Phe Arg Met Tyr Glu Lys Leu
               355                 360                 365
   gcc ggt atg acg ggt aca gct aag aca gag gaa gaa gaa ttc cgc aac     1692
   Ala Gly Met Thr Gly Thr Ala Lys Thr Glu Glu Glu Glu Phe Arg Asn
           370                 375                 380
   atc tac aac atg cag gtt gtc acg atc cct acc aac agg cct gtt gtc     1740
   Ile Tyr Asn Met Gln Val Val Thr Ile Pro Thr Asn Arg Pro Val Val
       385                 390                 395
   cgt gat gac cgc ccg gat tta att tac cgc acg atg gaa gga aag ttt     1788
   Arg Asp Asp Arg Pro Asp Leu Ile Tyr Arg Thr Met Glu Gly Lys Phe
   400                 405                 410                 415
   aag gca gtt gcg gag gat gtc gca cag cgt tac atg acg gga cag cct     1836
   Lys Ala Val Ala Glu Asp Val Ala Gln Arg Tyr Met Thr Gly Gln Pro
                   420                 425                 430
   gtt cta gtc ggt acg gtt gcc gtt gaa aca tct gaa ttg att tct aag     1884
   Val Leu Val Gly Thr Val Ala Val Glu Thr Ser Glu Leu Ile Ser Lys
               435                 440                 445
   ctg ctt aaa aac aaa gga att ccg cat caa gtg tta aat gcc aaa aac     1932
   Leu Leu Lys Asn Lys Gly Ile Pro His Gln Val Leu Asn Ala Lys Asn
           450                 455                 460
   cat gaa cgt gaa gcg cag atc att gaa gag gcc ggc caa aaa ggc gca     1980
   His Glu Arg Glu Ala Gln Ile Ile Glu Glu Ala Gly Gln Lys Gly Ala
       465                 470                 475
   gtt acg att gcg act aac atg gcg ggg cgc gga acg gac att aag ctt     2028
   Val Thr Ile Ala Thr Asn Met Ala Gly Arg Gly Thr Asp Ile Lys Leu
   480                 485                 490                 495
   ggc gaa ggt gta aaa gag ctt ggc ggg ctc gct gta gtc gga aca gaa     2076
   Gly Glu Gly Val Lys Glu Leu Gly Gly Leu Ala Val Val Gly Thr Glu
                   500                 505                 510
   cga cat gaa tca cgc cgg att gac aat cag ctt cga ggt cgt tcc gga     2124
   Arg His Glu Ser Arg Arg Ile Asp Asn Gln Leu Arg Gly Arg Ser Gly
               515                 520                 525
   cgt cag gga gac ccg ggg att act caa ttt tat ctt tct atg gaa gat    2172
   Arg Gln Gly Asp Pro Gly Ile Thr Gln Phe Tyr Leu Ser Met Glu Asp
           530                 535                 540
   gaa ttg atg cgc aga ttc gga gct gag cgg aca atg gcg atg ctt gac    2220
   Glu Leu Met Arg Arg Phe Gly Ala Glu Arg Thr Met Ala Met Leu Asp
       545                 550                 555
   cgc ttc ggc atg gac gac tct act cca atc caa agc aaa atg gta tct    2268
   Arg Phe Gly Met Asp Asp Ser Thr Pro Ile Gln Ser Lys Met Val Ser
   560                 565                 570                 575
   cgc gcg gtt gaa tcg tct caa aaa cgc gtc gaa ggc aat aac ttc gat    2316
   Arg Ala Val Glu Ser Ser Gln Lys Arg Val Glu Gly Asn Asn Phe Asp
                   580                 585                 590
   tcg cgt aaa cag ctt ctg caa tat gat gat gtt ctc cgc cag cag cgt    2364
   Ser Arg Lys Gln Leu Leu Gln Tyr Asp Asp Val Leu Arg Gln Gln Arg
               595                 600                 605
   gag gtc att tat aag cag cgc ttt gaa gtc att gac tct gaa aac ctg    2412
   Glu Val Ile Tyr Lys Gln Arg Phe Glu Val Ile Asp Ser Glu Asn Leu
           610                 615                 620
   cgt gaa atc gtt gaa aat atg atc aag tct tct ctc gaa cgc gca att    2460
   Arg Glu Ile Val Glu Asn Met Ile Lys Ser Ser Leu Glu Arg Ala Ile
       625                 630                 635
   gca gcc tat acg cca aga gaa gag ctt cct gag gag tgg aag ctt gac    2508
   Ala Ala Tyr Thr Pro Arg Glu Glu Leu Pro Glu Glu Trp Lys Leu Asp
   640                 645                 650                 655
   ggt cta gtt gat ctt atc aac aca act tat ctt gat gaa ggt gca ctt    2556
   Gly Leu Val Asp Leu Ile Asn Thr Thr Tyr Leu Asp Glu Gly Ala Leu
                   660                 665                 670
   gag aag agc gat atc ttc ggc aaa gaa ccg gat gaa atg ctt gag ctc    2604
   Glu Lys Ser Asp Ile Phe Gly Lys Glu Pro Asp Glu Met Leu Glu Leu
               675                 680                 685
   att atg gat cgc atc atc aca aaa tat aat gag aag gaa gag caa ttc    2652
   Ile Met Asp Arg Ile Ile Thr Lys Tyr Asn Glu Lys Glu Glu Gln Phe
           690                 695                 700
   ggc aaa gag caa atg cgc gaa ttc gaa aaa gtt atc gtt ctt cgt gcc    2700
   Gly Lys Glu Gln Met Arg Glu Phe Glu Lys Val Ile Val Leu Arg Ala
       705                 710                 715
   gtt gat tct aaa tgg atg gat cat att gat gcg atg gat cag ctc cgc    2748
   Val Asp Ser Lys Trp Met Asp His Ile Asp Ala Met Asp Gln Leu Arg
   720                 725                 730                 735
   caa ggg att cac ctt cgt gct tac gcg cag acg aac ccg ctt cgt gag    2796
   Gln Gly Ile His Leu Arg Ala Tyr Ala Gln Thr Asn Pro Leu Arg Glu
                   740                 745                 750
   tat caa atg gaa ggt ttt gcg atg ttt gag cat atg att gaa tca att    2844
   Tyr Gln Met Glu Gly Phe Ala Met Phe Glu His Met Ile Glu Ser Ile
               755                 760                 765
   gag gac gaa gtc gca aaa ttt gtg atg aaa gct gag att gaa aac aat    2892
   Glu Asp Glu Val Ala Lys Phe Val Met Lys Ala Glu Ile Glu Asn Asn
           770                 775                 780
   ctg gag cgt gaa gag gtt gta caa ggt caa aca aca gct cat cag ccg    2940
   Leu Glu Arg Glu Glu Val Val Gln Gly Gln Thr Thr Ala His Gln Pro
       785                 790                 795
   caa gaa ggc gac gat aac aaa aaa gca aag aaa gca ccg gtt cgc aaa    2988
   Gln Glu Gly Asp Asp Asn Lys Lys Ala Lys Lys Ala Pro Val Arg Lys
   800                 805                 810                 815
   gtg gtt gat atc gga cga aat gcc cca tgc cac tgc gga agc ggg aaa    3036
   Val Val Asp Ile Gly Arg Asn Ala Pro Cys His Cys Gly Ser Gly Lys
                   820                 825                 830
   aaa tat aaa aat tgc tgc ggc cgt act gaa tag ttcgccccgg caagtttact 3089
   Lys Tyr Lys Asn Cys Cys Gly Arg Thr Glu
               835                 840
   gaacgcggcg cctgcaggct gcgatctttt aatgaggtga atgaaatgaa ttatcagaaa 3149
   ttagagcaga gctcgaaaat atggcttctc gtttagcgga ctttaggggg tctctttgac 3209
   ctcgaatcaa aggaggcccg cattgctgag ctagatgaac aaatggctga tccggaattc 3269
   tggaatgatc agcaaaaagc tcaaacggtt ataaatgaag caaacggttt aaaggattat 3329
   gtcaattcgt ataaaaaatt gaatgaatcc cacgaagaat tacaaatgac tcatgatctt 3389
   ttgaaagaag agccggacac tgatctccag cttgagcttg aaaaagaact aaagtcatta 3449
   acaaaagagt tcaatgagtt tgagcttcag cttcttctca gcgagccgta tgataaaaat 3509
   aacgcgattt tagaactgca ccctggtgct ggcggtacag agtcacagga ctggggctct 3569
   atgcttctta gaatgtatac aagatgggga gagcgccgcg gctttaaagt agagactctc 3629
   gattaccttc caggtgacga ggcgggaatc aagtcagtga cattgctcat caaaggacac 3689
   aacgcttacg ggtatctcaa agcagaaaaa ggtgttcatc gtcttgtgcg gatctcacca 3749
   tttgattcat caggccgccg ccacacatct ttcgtttcat gtgaagtcat gcctgaattt 3809
   aacgatgaaa ttgatattga tattcgtacg gaggatatta aagttgacac gtaccgtgca 3869
   agcggcgcgg gcggacagca cgtcaatacg acggattcag ccgttcggat tactcacttg 3929
   ccgacgaacg tagttgtgac atgccaaacg gagcgctcac aaattaaaaa ccgtgaaaga 3989
   gccatgaaaa tgctgaaggc caagctgtat cagcgcagaa ttgaagagca gcaggcagag 4049
   ctggatgaaa ttcgcggtga acaaaaagaa atcggctggg gcagccaaat ccgttcttat 4109
   gtattccatc cgtattccat ggtaaaagac catcgggac                         4148
<210> 2
   <211> 841
   <212> PRT
   <213> Bacillus subtilis
<400> 2
   Met Leu Gly Ile Leu Asn Lys Met Phe Asp Pro Thr Lys Arg Thr Leu
   1               5                   10                  15
   Asn Arg Tyr Glu Lys Ile Ala Asn Asp Ile Asp Ala Ile Arg Gly Asp
               20                  25                  30
   Tyr Glu Asn Leu Ser Asp Asp Ala Leu Lys His Lys Thr Ile Glu Phe
           35                  40                  45
   Lys Glu Arg Leu Glu Lys Gly Ala Thr Thr Asp Asp Leu Leu Val Glu
       50                  55                  60
   Ala Phe Ala Val Val Arg Glu Ala Ser Arg Arg Val Thr Gly Met Phe
   65                  70                  75                  80
   Pro Phe Lys Val Gln Leu Met Gly Gly Val Ala Leu His Asp Gly Asn
                   85                  90                  95
   Ile Ala Glu Met Lys Thr Gly Glu Gly Lys Thr Leu Thr Ser Thr Leu
               100                 105                 110
   Pro Val Tyr Leu Asn Ala Leu Thr Gly Lys Gly Val His Val Val Thr
           115                 120                 125
   Val Asn Glu Tyr Leu Ala Ser Arg Asp Ala Glu Gln Met Gly Lys Ile
       130                 135                 140
   Phe Glu Phe Leu Gly Leu Thr Val Gly Leu Asn Leu Asn Ser Met Ser
   145                 150                 155                 160
   Lys Asp Glu Lys Arg Glu Ala Tyr Ala Ala Asp Ile Thr Tyr Ser Thr
                   165                 170                 175
   Asn Asn Glu Leu Gly Phe Asp Tyr Leu Arg Asp Asn Met Val Leu Tyr
               180                 185                 190
   Lys Glu Gln Met Val Gln Arg Pro Leu His Phe Ala Val Ile Asp Glu
           195                 200                 205
   Val Asp Ser Ile Leu Ile Asp Glu Ala Arg Thr Pro Leu Ile Ile Ser
       210                 215                 220
   Gly Gln Ala Ala Lys Ser Thr Lys Leu Tyr Val Gln Ala Asn Ala Phe
   225                 230                 235                 240
   Val Arg Thr Leu Lys Ala Glu Lys Asp Tyr Thr Tyr Asp Ile Lys Thr
                   245                 250                 255
   Lys Ala Val Gln Leu Thr Glu Glu Gly Met Thr Lys Ala Glu Lys Ala
               260                 265                 270
   Phe Gly Ile Asp Asn Leu Phe Asp Val Lys His Val Ala Leu Asn His
           275                 280                 285
   His Ile Asn Gln Ala Leu Lys Ala His Val Ala Met Gln Lys Asp Val
       290                 295                 300
   Asp Tyr Val Val Glu Asp Gly Gln Val Val Ile Val Asp Ser Phe Thr
   305                 310                 315                 320
   Gly Arg Leu Met Lys Gly Arg Arg Tyr Ser Glu Gly Leu His Gln Ala
                   325                 330                 335
   Ile Glu Ala Lys Glu Gly Leu Glu Ile Gln Asn Glu Ser Met Thr Leu
               340                 345                 350
   Ala Thr Ile Thr Phe Gln Asn Tyr Phe Arg Met Tyr Glu Lys Leu Ala
           355                 360                 365
   Gly Met Thr Gly Thr Ala Lys Thr Glu Glu Glu Glu Phe Arg Asn Ile
       370                 375                 380
   Tyr Asn Met Gln Val Val Thr Ile Pro Thr Asn Arg Pro Val Val Arg
   385                 390                 395                 400
   Asp Asp Arg Pro Asp Leu Ile Tyr Arg Thr Met Glu Gly Lys Phe Lys
                   405                 410                 415
   Ala Val Ala Glu Asp Val Ala Gln Arg Tyr Met Thr Gly Gln Pro Val
                   420                 425                 430
   Leu Val Gly Thr Val Ala Val Glu Thr Ser Glu Leu Ile Ser Lys Leu
           435                 440                 445
   Leu Lys Asn Lys Gly Ile Pro His Gln Val Leu Asn Ala Lys Asn His
       450                 455                 460
   Glu Arg Glu Ala Gln Ile Ile Glu Glu Ala Gly Gln Lys Gly Ala Val
   465                 470                 475                 480
   Thr Ile Ala Thr Asn Met Ala Gly Arg Gly Thr Asp Ile Lys Leu Gly
                   485                 490                 495
   Glu Gly Val Lys Glu Leu Gly Gly Leu Ala Val Val Gly Thr Glu Arg
               500                 505                 510
   His Glu Ser Arg Arg Ile Asp Asn Gln Leu Arg Gly Arg Ser Gly Arg
           515                 520                 525
   Gln Gly Asp Pro Gly Ile Thr Gln Phe Tyr Leu Ser Met Glu Asp Glu
       530                 535                 540
   Leu Met Arg Arg Phe Gly Ala Glu Arg Thr Met Ala Met Leu Asp Arg
   545                 550                 555                 560
   Phe Gly Met Asp Asp Ser Thr Pro Ile Gln Ser Lys Met Val Ser Arg
                   565                 570                 575
   Ala Val Glu Ser Ser Gln Lys Arg Val Glu Gly Asn Asn Phe Asp Ser
               580                 585                 590
   Arg Lys Gln Leu Leu Gln Tyr Asp Asp Val Leu Arg Gln Gln Arg Glu
           595                 600                 605
   Val Ile Tyr Lys Gln Arg Phe Glu Val Ile Asp Ser Glu Asn Leu Arg
       610                 615                 620
   Glu Ile Val Glu Asn Met Ile Lys Ser Ser Leu Glu Arg Ala Ile Ala
   625                 630                 635                 640
   Ala Tyr Thr Pro Arg Glu Glu Leu Pro Glu Glu Trp Lys Leu Asp Gly
                   645                 650                 655
   Leu Val Asp Leu Ile Asn Thr Thr Tyr Leu Asp Glu Gly Ala Leu Glu
               660                 665                 670
   Lys Ser Asp Ile Phe Gly Lys Glu Pro Asp Glu Met Leu Glu Leu Ile
           675                 680                 685
   Met Asp Arg Ile Ile Thr Lys Tyr Asn Glu Lys Glu Glu Gln Phe Gly
       690                 695                 700
   Lys Glu Gln Met Arg Glu Phe Glu Lys Val Ile Val Leu Arg Ala Val
   705                 710                 715                 720
   Asp Ser Lys Trp Met Asp His Ile Asp Ala Met Asp Gln Leu Arg Gln
                   725                 730                 735
   Gly Ile His Leu Arg Ala Tyr Ala Gln Thr Asn Pro Leu Arg Glu Tyr
               740                 745                 750
   Gln Met Glu Gly Phe Ala Met Phe Glu His Met Ile Glu Ser Ile Glu
           755                 760                 765
   Asp Glu Val Ala Lys Phe Val Met Lys Ala Glu Ile Glu Asn Asn Leu
       770                 775                 780
   Glu Arg Glu Glu Val Val Gln Gly Gln Thr Thr Ala His Gln Pro Gln
   785                 790                 795                 800
   Glu Gly Asp Asp Asn Lys Lys Ala Lys Lys Ala Pro Val Arg Lys Val
                   805                 810                 815
   Val Asp Ile Gly Arg Asn Ala Pro Cys His Cys Gly Ser Gly Lys Lys
               820                 825                 830
   Tyr Lys Asn Cys Cys Gly Arg Thr Glu
           835                 840
<210> 3
   <211> 2706
   <212> DNA
   <213> Escherichia coli
<220>
   <221> CDS
   <222> (1)..(2706)
<400> 3
   atg cta atc aaa tta tta act aaa gtt ttc ggt agt cgt aac gat cgc      48
   Met Leu Ile Lys Leu Leu Thr Lys Val Phe Gly Ser Arg Asn Asp Arg
   1               5                   10                  15
   acc ctg cgc cgg atg cgc aaa gtg gtc aac atc atc aat gcc atg gaa      96
   Thr Leu Arg Arg Met Arg Lys Val Val Asn Ile Ile Asn Ala Met Glu
               20                  25                  30
   ccg gag atg gaa aaa ctc tcc gac gaa gaa ctg aaa ggg aaa acc gca      144
   Pro Glu Met Glu Lys Leu Ser Asp Glu Glu Leu Lys Gly Lys Thr Ala
           35                  40                  45
   gag ttt cgt gcg cgt ctg gaa aaa ggc gaa gtg ctg gaa aat ctg atc      192
   Glu Phe Arg Ala Arg Leu Glu Lys Gly Glu Val Leu Glu Asn Leu Ile
       50                  55                  60
   ccg gaa gct ttc gcc gtg gtg cgt gag gca agt aag cgc gtc ttt ggt      240
   Pro Glu Ala Phe Ala Val Val Arg Glu Ala Ser Lys Arg Val Phe Gly
   65                  70                  75                  80
   atg cgt cac ttc gac gtt cag tta ctc ggc ggt atg gtt ctt aac gaa      288
   Met Arg His Phe Asp Val Gln Leu Leu Gly Gly Met Val Leu Asn Glu
                   85                  90                  95
   cgc tgc atc gcc gaa atg cgt acc ggt gaa ggt aaa acc ctg acc gca      336
   Arg Cys Ile Ala Glu Met Arg Thr Gly Glu Gly Lys Thr Leu Thr Ala
               100                 105                 110
   acg ctg cct gct tac ctg aac gca cta acc ggt aaa ggc gta cac gta      384
   Thr Leu Pro Ala Tyr Leu Asn Ala Leu Thr Gly Lys Gly Val His Val
           115                 120                 125
   gtt acc gtc aac gac tac ctg gcg caa cgt gac gcc gaa aac aac cgt      432
   Val Thr Val Asn Asp Tyr Leu Ala Gln Arg Asp Ala Glu Asn Asn Arg
       130                 135                 140
   ccg ctg ttt gaa ttc ctt ggc ctg act gtc ggt atc aac ctg ccg ggc      480
   Pro Leu Phe Glu Phe Leu Gly Leu Thr Val Gly Ile Asn Leu Pro Gly
   145                 150                 155                 160
   atg cca gca ccg gca aag cgt gaa gcc tac gct gct gac atc act tac      528
   Met Pro Ala Pro Ala Lys Arg Glu Ala Tyr Ala Ala Asp Ile Thr Tyr
                   165                 170                 175
   ggt acg aac aac gaa tac ggc ttt gac tac ctg cgc gac aac atg gca      576
   Gly Thr Asn Asn Glu Tyr Gly Phe Asp Tyr Leu Arg Asp Asn Met Ala
               180                 185                 190
   ttc agc cct gaa gaa cgt gta caa cgt aaa ctg cac tat gcg ctg gtg      624
   Phe Ser Pro Glu Glu Arg Val Gln Arg Lys Leu His Tyr Ala Leu Val
           195                 200                 205
   gac gaa gtg gac tcc atc ctc atc gat gaa gcg cgt aca ccg ctg atc    672
   Asp Glu Val Asp Ser Ile Leu Ile Asp Glu Ala Arg Thr Pro Leu Ile
       210                 215                 220
   att tcc ggc cca gca gaa gac agc tcg gaa atg tat aaa cgc gtg aat    720
   Ile Ser Gly Pro Ala Glu Asp Ser Ser Glu Met Tyr Lys Arg Val Asn
   225                 230                 235                 240
   aaa att att ccg cac ctg atc cgt cag gaa aaa gaa gac tcc gaa acc    768
   Lys Ile Ile Pro His Leu Ile Arg Gln Glu Lys Glu Asp Ser Glu Thr
                   245                 250                 255
   ttc cag ggc gaa ggc cac ttc tcg gtg gat gaa aaa tct cgc cag gtg    816
   Phe Gln Gly Glu Gly His Phe Ser Val Asp Glu Lys Ser Arg Gln Val
               260                 265                 270
   aac ctg acc gaa cgt ggt ctg gtt ctg att gaa gaa ctg ctg gtt aaa    864
   Asn Leu Thr Glu Arg Gly Leu Val Leu Ile Glu Glu Leu Leu Val Lys
           275                 280                 285
   gaa ggc atc atg gat gaa ggt gag tct ctg tac tct ccg gcc aac atc    912
   Glu Gly Ile Met Asp Glu Gly Glu Ser Leu Tyr Ser Pro Ala Asn Ile
       290                 295                 300
   atg ctg atg cac cac gta acg gcg gcg ctg cgc gct cat gcg ctg ttt    960
   Met Leu Met His His Val Thr Ala Ala Leu Arg Ala His Ala Leu Phe
   305                 310                 315                 320
   acc cgc gac gtc gac tac atc gtt aaa gat ggt gaa gtt atc atc gtt    1008
   Thr Arg Asp Val Asp Tyr Ile Val Lys Asp Gly Glu Val Ile Ile Val
                   325                 330                 335
   gac gaa cac acc ggt cgt acc atg cag ggc cgt cgc tgg tcc gat ggt    1056
   Asp Glu His Thr Gly Arg Thr Met Gln Gly Arg Arg Trp Ser Asp Gly
               340                 345                 350
   ctg cac cag gct gtg gaa gcg aaa gaa ggt gtg cag atc cag aac gaa    1104
   Leu His Gln Ala Val Glu Ala Lys Glu Gly Val Gln Ile Gln Asn Glu
           355                 360                 365
   aac cag acg ctg gct tcg atc acc ttc cag aac tac ttc cgt ctg tat    1152
   Asn Gln Thr Leu Ala Ser Ile Thr Phe Gln Asn Tyr Phe Arg Leu Tyr
       370                 375                 380
   gaa aaa ctg gcg ggg atg act ggt act gct gat acc gaa gct ttc gaa    1200
   Glu Lys Leu Ala Gly Met Thr Gly Thr Ala Asp Thr Glu Ala Phe Glu
   385                 390                 395                 400
   ttc agc tcc atc tat aag ctg gat act gtc gtt gtt ccg acc aac cgt    1248
   Phe Ser Ser Ile Tyr Lys Leu Asp Thr Val Val Val Pro Thr Asn Arg
                   405                 410                 415
   cca atg att cgt aaa gat ctg ccg gac ctg gtc tac atg act gaa gcg    1296
   Pro Met Ile Arg Lys Asp Leu Pro Asp Leu Val Tyr Met Thr Glu Ala
               420                 425                 430
   gaa aaa att cag gcg atc att gaa gat atc aaa gaa cgt act gcg aaa    1344
   Glu Lys Ile Gln Ala Ile Ile Glu Asp Ile Lys Glu Arg Thr Ala Lys
           435                 440                 445
   ggc cag ccg gtg ctg gtg ggt aca atc tcc atc gaa aaa tcg gag ctg    1392
   Gly Gln Pro Val Leu Val Gly Thr Ile Ser Ile Glu Lys Ser Glu Leu
       450                 455                 460
   gtg tca aat gaa ctg acc aaa gcc ggt att aag cac aac gtc ctg aac    1440
   Val Ser Asn Glu Leu Thr Lys Ala Gly Ile Lys His Asn Val Leu Asn
   465                 470                 475                 480
   gcc aaa ttc cat gcc aac gaa gcg gcg att gtt gct cag gca ggt tat    1488
   Ala Lys Phe His Ala Asn Glu Ala Ala Ile Val Ala Gln Ala Gly Tyr
                   485                 490                 495
   ccg gct gcg gtg act atc gcg acc aac atg gcg ggt cgt ggt acc gat    1536
   Pro Ala Ala Val Thr Ile Ala Thr Asn Met Ala Gly Arg Gly Thr Asp
               500                 505                 510
   att gtg ctc ggt ggt agc tgg cag gca gaa gtt gcc gcg ctg gaa aat    1584
   Ile Val Leu Gly Gly Ser Trp Gln Ala Glu Val Ala Ala Leu Glu Asn
           515                 520                 525
   ccg act gca gag caa att gaa aaa att aaa gcc gac tgg cag gta cgt    1632
   Pro Thr Ala Glu Gln Ile Glu Lys Ile Lys Ala Asp Trp Gln Val Arg
       530                 535                 540
   cac gat gcg gta ctg gca gca ggt ggc ctg cat atc atc ggt act gaa    1680
   His Asp Ala Val Leu Ala Ala Gly Gly Leu His Ile Ile Gly Thr Glu
   545                 550                 555                 560
   cgt cac gaa tcc cgt cgt atc gat aac cag ctg cgc ggt cgt tct ggt    1728
   Arg His Glu Ser Arg Arg Ile Asp Asn Gln Leu Arg Gly Arg Ser Gly
                   565                 570                 575
   cgt cag ggg gat gct ggt tct tct cgt ttc tac ctg tcg atg gaa gat    1776
   Arg Gln Gly Asp Ala Gly Ser Ser Arg Phe Tyr Leu Ser Met Glu Asp
               580                 585                 590
   gcg ctg atg cgt att ttt gct tcc gac cga gta tcc ggc atg atg cgt    1824
   Ala Leu Met Arg Ile Phe Ala Ser Asp Arg Val Ser Gly Met Met Arg
           595                 600                 605
   aaa ctg ggt atg aag cca ggc gaa gcc att gag cac ccg tgg gtg acc    1872
   Lys Leu Gly Met Lys Pro Gly Glu Ala Ile Glu His Pro Trp Val Thr
       610                 615                 620
   aaa gcg att gcc aac gcc cag cgt aaa gtt gaa agc cgt aac ttc gac    1920
   Lys Ala Ile Ala Asn Ala Gln Arg Lys Val Glu Ser Arg Asn Phe Asp
   625                 630                 635                 640
   att cgt aag caa ctg ctg gaa tat gat gac gtg gct aac gat cag cgt    1968
   Ile Arg Lys Gln Leu Leu Glu Tyr Asp Asp Val Ala Asn Asp Gln Arg
                   645                 650                 655
   cgc gcc att tac tcc cag cgt aac gaa ctg ctg gat gtc agc gat gtg    2016
   Arg Ala Ile Tyr Ser Gln Arg Asn Glu Leu Leu Asp Val Ser Asp Val
               660                 665                 670
   agc gaa acc atc aac agc att cgt gaa gat gtg ttc aaa gcg acc att    2064
   Ser Glu Thr Ile Asn Ser Ile Arg Glu Asp Val Phe Lys Ala Thr Ile
               675                 680                 685
   gat gcc tac att ccg cca cag tcg ctg gaa gaa atg tgg gat att ccg     2112
   Asp Ala Tyr Ile Pro Pro Gln Ser Leu Glu Glu Met Trp Asp Ile Pro
       690                 695                 700
   ggg ctg cag gaa cgt ctg aag aac gat ttc gac ctc gat ttg cca att     2160
   Gly Leu Gln Glu Arg Leu Lys Asn Asp Phe Asp Leu Asp Leu Pro Ile
   705                 710                 715                 720
   gcc gag tgg ctg gat aaa gaa cca gaa ctg cat gaa gag acg ctg cgt     2208
   Ala Glu Trp Leu Asp Lys Glu Pro Glu Leu His Glu Glu Thr Leu Arg
                   725                 730                 735
   gag cgc att ctg gcg cag tcc atc gaa gtg tat cag cgt aaa gaa gaa     2256
   Glu Arg Ile Leu Ala Gln Ser Ile Glu Val Tyr Gln Arg Lys Glu Glu
               740                 745                 750
   gtg gtt ggt gct gag atg atg cgt cac ttc gag aaa ggc gtc atg ctg     2304
   Val Val Gly Ala Glu Met Met Arg His Phe Glu Lys Gly Val Met Leu
           755                 760                 765
   caa act ctc gac tct ctg tgg aaa gag cac ctg gca gcg atg gac tat     2352
   Gln Thr Leu Asp Ser Leu Trp Lys Glu His Leu Ala Ala Met Asp Tyr
       770                 775                 780
   ctg cgt cag ggt atc cac ctg cgt ggc tat gca cag aaa gat ccg aag     2400
   Leu Arg Gln Gly Ile His Leu Arg Gly Tyr Ala Gln Lys Asp Pro Lys
   785                 790                 795                 800
   cag gaa tac aaa cgt gaa tcg ttc tcc atg ttt gca gcg atg ctg gag     2448
   Gln Glu Tyr Lys Arg Glu Ser Phe Ser Met Phe Ala Ala Met Leu Glu
                   805                 810                 815
   tcg ttg aaa tat gaa gtt atc agt acg ctg agc aaa gtt cag gta cgt     2496
   Ser Leu Lys Tyr Glu Val Ile Ser Thr Leu Ser Lys Val Gln Val Arg
               820                 825                 830
   atg cct gaa gag gtt gag gag ctg gaa caa cag cgt cgt atg gaa gcc     2544
   Met Pro Glu Glu Val Glu Glu Leu Glu Gln Gln Arg Arg Met Glu Ala
           835                 840                 845
   gag cgt tta gcg caa atg cag cag ctt agc cat cag gat gac gac tct     2592
   Glu Arg Leu Ala Gln Met Gln Gln Leu Ser His Gln Asp Asp Asp Ser
       850                 855                 860
   gca gcc gca gct gca ctg gcg gcg caa acc ggt gaa cgc aaa gta gga     2640
   Ala Ala Ala Ala Ala Leu Ala Ala Gln Thr Gly Glu Arg Lys Val Gly
   865                 870                 875                 880
   cgt aac gat cct tgc ccg tgt ggt tct ggt aaa aaa tac aag cag tgc     2688
   Arg Asn Asp Pro Cys Pro Cys Gly Ser Gly Lys Lys Tyr Lys Gln Cys
                   885                 890                 895
   cat ggc cgc ctg caa taa                                             2706
   His Gly Arg Leu Gln
               900
<210> 4
   <211> 901
   <212> PRT
   <213> Escherichia coli
<400> 4
   Met Leu Ile Lys Leu Leu Thr Lys Val Phe Gly Ser Arg Asn Asp Arg
    1               5                  10                  15
   Thr Leu Arg Arg Met Arg Lys Val Val Asn Ile Ile Asn Ala Met Glu
               20                  25                  30
   Pro Glu Met Glu Lys Leu Ser Asp Glu Glu Leu Lys Gly Lys Thr Ala
           35                  40                  45
   Glu Phe Arg Ala Arg Leu Glu Lys Gly Glu Val Leu Glu Asn Leu Ile
       50                  55                  60
   Pro Glu Ala Phe Ala Val Val Arg Glu Ala Ser Lys Arg Val Phe Gly
   65                  70                  75                  80
   Met Arg His Phe Asp Val Gln Leu Leu Gly Gly Met Val Leu Asn Glu
                   85                  90                  95
   Arg Cys Ile Ala Glu Met Arg Thr Gly Glu Gly Lys Thr Leu Thr Ala
               100                 105                 110
   Thr Leu Pro Ala Tyr Leu Asn Ala Leu Thr Gly Lys Gly Val His Val
           115                 120                 125
   Val Thr Val Asn Asp Tyr Leu Ala Gln Arg Asp Ala Glu Asn Asn Arg
       130                 135                 140
   Pro Leu Phe Glu Phe Leu Gly Leu Thr Val Gly Ile Asn Leu Pro Gly
   145                 150                 155                 160
   Met Pro Ala Pro Ala Lys Arg Glu Ala Tyr Ala Ala Asp Ile Thr Tyr
                   165                 170                 175
   Gly Thr Asn Asn Glu Tyr Gly Phe Asp Tyr Leu Arg Asp Asn Met Ala
               180                 185                 190
   Phe Ser Pro Glu Glu Arg Val Gln Arg Lys Leu His Tyr Ala Leu Val
           195                 200                 205
   Asp Glu Val Asp Ser Ile Leu Ile Asp Glu Ala Arg Thr Pro Leu Ile
       210                 215                 220
   Ile Ser Gly Pro Ala Glu Asp Ser Ser Glu Met Tyr Lys Arg Val Asn
   225                 230                 235                 240
   Lys Ile Ile Pro His Leu Ile Arg Gln Glu Lys Glu Asp Ser Glu Thr
                   245                 250                 255
   Phe Gln Gly Glu Gly His Phe Ser Val Asp Glu Lys Ser Arg Gln Val
               260                 265                 270
   Asn Leu Thr Glu Arg Gly Leu Val Leu Ile Glu Glu Leu Leu Val Lys
           275                 280                 285
   Glu Gly Ile Met Asp Glu Gly Glu Ser Leu Tyr Ser Pro Ala Asn Ile
       290                 295                 300
   Met Leu Met His His Val Thr Ala Ala Leu Arg Ala His Ala Leu Phe
   305                 310                 315                 320
   Thr Arg Asp Val Asp Tyr Ile Val Lys Asp Gly Glu Val Ile Ile Val
                   325                 330                 335
   Asp Glu His Thr Gly Arg Thr Met Gln Gly Arg Arg Trp Ser Asp Gly
               340                 345                 350
   Leu His Gln Ala Val Glu Ala Lys Glu Gly Val Gln Ile Gln Asn Glu
           355                 360                 365
   Asn Gln Thr Leu Ala Ser Ile Thr Phe Gln Asn Tyr Phe Arg Leu Tyr
       370                 375                 380
   Glu Lys Leu Ala Gly Met Thr Gly Thr Ala Asp Thr Glu Ala Phe Glu
   385                 390                 395                 400
   Phe Ser Ser Ile Tyr Lys Leu Asp Thr Val Val Val Pro Thr Asn Arg
                   405                 410                 415
   Pro Met Ile Arg Lys Asp Leu Pro Asp Leu Val Tyr Met Thr Glu Ala
                   420                 425                 430
   Glu Lys Ile Gln Ala Ile Ile Glu Asp Ile Lys Glu Arg Thr Ala Lys
               435                 440                 445
   Gly Gln Pro Val Leu Val Gly Thr Ile Ser Ile Glu Lys Ser Glu Leu
           450                 455                 460
   Val Ser Asn Glu Leu Thr Lys Ala Gly Ile Lys His Asn Val Leu Asn
   465                 470                 475                 480
   Ala Lys Phe His Ala Asn Glu Ala Ala Ile Val Ala Gln Ala Gly Tyr
                   485                 490                 495
   Pro Ala Ala Val Thr Ile Ala Thr Asn Met Ala Gly Arg Gly Thr Asp
               500                 505                 510
   Ile Val Leu Gly Gly Ser Trp Gln Ala Glu Val Ala Ala Leu Glu Asn
           515                 520                 525
   Pro Thr Ala Glu Gln Ile Glu Lys Ile Lys Ala Asp Trp Gln Val Arg
       530                 535                 540
   His Asp Ala Val Leu Ala Ala Gly Gly Leu His Ile Ile Gly Thr Glu
   545                 550                 555                 560
   Arg His Glu Ser Arg Arg Ile Asp Asn Gln Leu Arg Gly Arg Ser Gly
                   565                 570                 575
   Arg Gln Gly Asp Ala Gly Ser Ser Arg Phe Tyr Leu Ser Met Glu Asp
               580                 585                 590
   Ala Leu Met Arg Ile Phe Ala Ser Asp Arg Val Ser Gly Met Met Arg
           595                 600                 605
   Lys Leu Gly Met Lys Pro Gly Glu Ala Ile Glu His Pro Trp Val Thr
       610                 615                 620
   Lys Ala Ile Ala Asn Ala Gln Arg Lys Val Glu Ser Arg Asn Phe Asp
   625                 630                 635                 640
   Ile Arg Lys Gln Leu Leu Glu Tyr Asp Asp Val Ala Asn Asp Gln Arg
                   645                 650                 655
   Arg Ala Ile Tyr Ser Gln Arg Asn Glu Leu Leu Asp Val Ser Asp Val
               660                 665                 670
   Ser Glu Thr Ile Asn Ser Ile Arg Glu Asp Val Phe Lys Ala Thr Ile
           675                 680                 685
   Asp Ala Tyr Ile Pro Pro Gln Ser Leu Glu Glu Met Trp Asp Ile Pro
       690                 695                 700
   Gly Leu Gln Glu Arg Leu Lys Asn Asp Phe Asp Leu Asp Leu Pro Ile
   705                 710                 715                 720
   Ala Glu Trp Leu Asp Lys Glu Pro Glu Leu His Glu Glu Thr Leu Arg
                   725                 730                 735
   Glu Arg Ile Leu Ala Gln Ser Ile Glu Val Tyr Gln Arg Lys Glu Glu
               740                 745                 750
   Val Val Gly Ala Glu Met Met Arg His Phe Glu Lys Gly Val Met Leu
           755                 760                 765
   Gln Thr Leu Asp Ser Leu Trp Lys Glu His Leu Ala Ala Met Asp Tyr
       770                 775                 780
   Leu Arg Gln Gly Ile His Leu Arg Gly Tyr Ala Gln Lys Asp Pro Lys
   785                 790                 795                 800
   Gln Glu Tyr Lys Arg Glu Ser Phe Ser Met Phe Ala Ala Met Leu Glu
                   805                 810                 815
   Ser Leu Lys Tyr Glu Val Ile Ser Thr Leu Ser Lys Val Gln Val Arg
               820                 825                 830
   Met Pro Glu Glu Val Glu Glu Leu Glu Gln Gln Arg Arg Met Glu Ala
           835                 840                 845
   Glu Arg Leu Ala Gln Met Gln Gln Leu Ser His Gln Asp Asp Asp Ser
       850                 855                 860
   Ala Ala Ala Ala Ala Leu Ala Ala Gln Thr Gly Glu Arg Lys Val Gly
   865                 870                 875                 880
   Arg Asn Asp Pro Cys Pro Cys Gly Ser Gly Lys Lys Tyr Lys Gln Cys
                   885                 890                 895
   His Gly Arg Leu Gln
   900
<210> 5
   <211> 2706
   <212> DNA
   <213> Bacillus licheniformis
<220>
   <221> CDS
   <222> (154)..(2679)
<400> 5
   gatccccctc ccggatcttc cgcagagggg attttttccg ttcccccgcg gtaaattgtt 60
   tggaaatgac aaaaggtatg atatgatatt gcatatataa aaattactgt ttactcatgc 120
   ttaaacaagg aaattaaaga ggagcgttat tct atg ctt gga att tta aat aaa  174
                                        Met Leu Gly Ile Leu Asn Lys
                                        1               5
   gtg ttt gat ccg aca aaa cgc acg ctc agc cgt tat gaa aag aaa gcg   222
   Val Phe Asp Pro Thr Lys Arg Thr Leu Ser Arg Tyr Glu Lys Lys Ala
           10                  15                  20
   aac gag att gat gcg ctc aag gca gat ata gag aag ctt tca gac gaa   270
   Asn Glu Ile Asp Ala Leu Lys Ala Asp Ile Glu Lys Leu Ser Asp Glu
       25                  30                  35
   gct ttg aag caa aag acg atc gag ttc aaa gag cgc ctt gaa aaa ggc   318
   Ala Leu Lys Gln Lys Thr Ile Glu Phe Lys Glu Arg Leu Glu Lys Gly
   40                  45                  50                  55
   gaa acg gtt gac gat ctt ttg gtt gaa gcg ttt gcc gtt gtc agg gaa   366
   Glu Thr Val Asp Asp Leu Leu Val Glu Ala Phe Ala Val Val Arg Glu
                   60                  65                  70
   gct tcc cgg cgc gtg aca ggc atg ttt ccg ttt aag gtt cag ctg atg   414
   Ala Ser Arg Arg Val Thr Gly Met Phe Pro Phe Lys Val Gln Leu Met
               75                  80                  85
   ggg ggc gtc gcc ctt cat gaa ggg aat atc gcc gaa atg aaa acg ggg   462
   Gly Gly Val Ala Leu His Glu Gly Asn Ile Ala Glu Met Lys Thr Gly
           90                  95                  100
   gaa ggt aaa acg ctg act tcc aca atg ccc gtt tac ttg aac gct ctg   510
   Glu Gly Lys Thr Leu Thr Ser Thr Met Pro Val Tyr Leu Asn Ala Leu
       105                 110                 115
   tca ggg aaa ggc gtt cac gtc gtg acg gtc aac gaa tac ctg gcg agc   558
   Ser Gly Lys Gly Val His Val Val Thr Val Asn Glu Tyr Leu Ala Ser
   120                 125                 130                 135
   cgc gac gct gaa gag atg ggg aaa atc ttt gag ttt ctc ggg ctg acg   606
   Arg Asp Ala Glu Glu Met Gly Lys Ile Phe Glu Phe Leu Gly Leu Thr
                   140                 145                 150
   gtc ggc cta aac ctg aac agc ctg tca aaa gac gag aag cgt gaa gcc    654
   Val Gly Leu Asn Leu Asn Ser Leu Ser Lys Asp Glu Lys Arg Glu Ala
               155                 160                 165
   tat gca gca gat att acg tat tct acg aat aat gag ctt ggc ttt gac    702
   Tyr Ala Ala Asp Ile Thr Tyr Ser Thr Asn Asn Glu Leu Gly Phe Asp
           170                 175                 180
   tac ttg cgc gac aac atg gtg ctt tat aaa gag cag atg gtt cag cgc    750
   Tyr Leu Arg Asp Asn Met Val Leu Tyr Lys Glu Gln Met Val Gln Arg
       185                 190                 195
   ccg ctt cat ttt gcg gtc atc gat gaa gtc gac tcc att ttg atc gat    798
   Pro Leu His Phe Ala Val Ile Asp Glu Val Asp Ser Ile Leu Ile Asp
   200                 205                 210                 215
   gaa gca aga acg ccg ctc atc att tct gga caa gcg gcc aaa tcc acc    846
   Glu Ala Arg Thr Pro Leu Ile Ile Ser Gly Gln Ala Ala Lys Ser Thr
                   220                 225                 230
   aag ctt tat gtt cag gcc aat gcg ttt gtc cgc acg cta aaa gcg gat    894
   Lys Leu Tyr Val Gln Ala Asn Ala Phe Val Arg Thr Leu Lys Ala Asp
               235                 240                 245
   cag gac tac aca tac gat gtg aaa aca aaa ggc gtt cag ctg act gaa    942
   Gln Asp Tyr Thr Tyr Asp Val Lys Thr Lys Gly Val Gln Leu Thr Glu
           250                 255                 260
   gag ggg atg aca aaa gct gaa aag gca ttt ggc atc gaa aac ttg ttt    990
   Glu Gly Met Thr Lys Ala Glu Lys Ala Phe Gly Ile Glu Asn Leu Phe
       265                 270                 275
   gac gtc cgc cat gtc gcc tta aac cat cat att gcc cag gcg ctg aaa    1038
   Asp Val Arg His Val Ala Leu Asn His His Ile Ala Gln Ala Leu Lys
   280                 285                 290                 295
   gcc cat gcg gcg atg cat aaa gac gtc gac tac gtc gtc gaa gac ggt    1086
   Ala His Ala Ala Met His Lys Asp Val Asp Tyr Val Val Glu Asp Gly
                   300                 305                 310
   cag gtc gtt atc gtc gac tct ttt aca ggc cgt ttg atg aaa ggc cgc    1134
   Gln Val Val Ile Val Asp Ser Phe Thr Gly Arg Leu Met Lys Gly Arg
               315                 320                 325
   cgc tac agc gac gga ctt cac cag gcc att gaa gcg aag gaa ggc ctt    1182
   Arg Tyr Ser Asp Gly Leu His Gln Ala Ile Glu Ala Lys Glu Gly Leu
           330                 335                 340
   gag atc caa aat gag agc atg acg ctc gcg acg atc acc ttc cag aac    1230
   Glu Ile Gln Asn Glu Ser Met Thr Leu Ala Thr Ile Thr Phe Gln Asn
       345                 350                 355
   tat ttc cga atg tat gaa aaa ttg gct gga atg acg ggt acc gca aaa    1278
   Tyr Phe Arg Met Tyr Glu Lys Leu Ala Gly Met Thr Gly Thr Ala Lys
   360                 365                 370                 375
   acg gaa gaa gaa gaa ttc cgc aac atc tac aac atg cag gtt gtt acg    1326
   Thr Glu Glu Glu Glu Phe Arg Asn Ile Tyr Asn Met Gln Val Val Thr
                   380                 385                 390
   att ccg acc aac aag ccg att gcc cgc gat gac cga ccg gat tta att    1374
   Ile Pro Thr Asn Lys Pro Ile Ala Arg Asp Asp Arg Pro Asp Leu Ile
               395                 400                 405
   tac cgg acc atg gaa gga aaa ttt aaa gct gtt gca gag gat gtc gcc    1422
   Tyr Arg Thr Met Glu Gly Lys Phe Lys Ala Val Ala Glu Asp Val Ala
           410                 415                 420
   cag cgc tat atg gtc gga cag ccg gta ctt gtc ggt acg gtt gcg gtt    1470
   Gln Arg Tyr Met Val Gly Gln Pro Val Leu Val Gly Thr Val Ala Val
       425                 430                 435
   gaa aca tct gaa ttg ata tca agg ctc ctt aaa aat aaa gga atc ccg    1518
   Glu Thr Ser Glu Leu Ile Ser Arg Leu Leu Lys Asn Lys Gly Ile Pro
   440                 445                 450                 455
   cat caa gtg ttg aac gcg aaa aac cat gag cgg gaa gct cag att atc    1566
   His Gln Val Leu Asn Ala Lys Asn His Glu Arg Glu Ala Gln Ile Ile
                   460                 465                 470
   gaa gat gcc ggg caa aaa ggc gcg gtc acc atc gcg acc aac atg gcg    1614
   Glu Asp Ala Gly Gln Lys Gly Ala Val Thr Ile Ala Thr Asn Met Ala
               475                 480                 485
   ggc cgc gga acg gac atc aag ctt ggc gaa ggt gta aaa gag ctt ggc    1662
   Gly Arg Gly Thr Asp Ile Lys Leu Gly Glu Gly Val Lys Glu Leu Gly
           490                 495                 500
   gga ctg gcc gtc atc ggt acg gaa cgc cat gaa tca agg cgg att gac    1710
   Gly Leu Ala Val Ile Gly Thr Glu Arg His Glu Ser Arg Arg Ile Asp
       505                 510                 515
   aac cag ctg cgc gga cgt tca ggc cgt cag ggg gac cct ggt atc acc    1758
   Asn Gln Leu Arg Gly Arg Ser Gly Arg Gln Gly Asp Pro Gly Ile Thr
   520                 525                 530                 535
   caa ttt tat ctg tcc atg gaa gat gaa tta atg aaa cgc ttc ggc gca    1806
   Gln Phe Tyr Leu Ser Met Glu Asp Glu Leu Met Lys Arg Phe Gly Ala
                   540                 545                 550
   gag cgg acg atg gcg atg ctt gac cgc ttc gga atg gac gat tcg acg    1854
   Glu Arg Thr Met Ala Met Leu Asp Arg Phe Gly Met Asp Asp Ser Thr
               555                 560                 565
   ccg ata cag agc aag atg gtt tca aga gcg gtc gaa tct tca cag aag    1902
   Pro Ile Gln Ser Lys Met Val Ser Arg Ala Val Glu Ser Ser Gln Lys
           570                 575                 580
   cgt gtg gaa ggc aac aac ttt gat gcc cgt aag cag ctt ctg caa tac    1950
   Arg Val Glu Gly Asn Asn Phe Asp Ala Arg Lys Gln Leu Leu Gln Tyr
       585                 590                 595
   gat gac gtg ctc cgc cag cag cgc gaa gtc atc tat aaa cag cgc ttt    1998
   Asp Asp Val Leu Arg Gln Gln Arg Glu Val Ile Tyr Lys Gln Arg Phe
   600                 605                 610                 615
   gag gtc atc gat tcc gat aac ctc cgc tcc atc gtc gaa aat atg att    2046
   Glu Val Ile Asp Ser Asp Asn Leu Arg Ser Ile Val Glu Asn Met Ile
                       620                 625                 630
   aaa gct tca ctc gag cgg gct gtt gct tca tat acg ccg aag gaa gat    2094
   Lys Ala Ser Leu Glu Arg Ala Val Ala Ser Tyr Thr Pro Lys Glu Asp
               635                 640                 645
   ctg cct gaa gag tgg aat ctt gac ggc ctt gtg gag ctt gta aat gcg    2142
   Leu Pro Glu Glu Trp Asn Leu Asp Gly Leu Val Glu Leu Val Asn Ala
           650                 655                 660
   aat ttc ctt gat gaa ggt gga gtg gag aaa agc gac att ttc gga aaa    2190
   Asn Phe Leu Asp Glu Gly Gly Val Glu Lys Ser Asp Ile Phe Gly Lys
       665                 670                 675
   gag ccc gag gag att aca gag ctc att tac gac cgc atc aaa acg aaa    2238
   Glu Pro Glu Glu Ile Thr Glu Leu Ile Tyr Asp Arg Ile Lys Thr Lys
   680                 685                 690                 695
   tac gat gag aaa gaa gag cgg tac ggc tct gaa caa atg cgc gaa ttt    2286
   Tyr Asp Glu Lys Glu Glu Arg Tyr Gly Ser Glu Gln Met Arg Glu Phe
                   700                 705                 710
   gag aaa gtc atc gtt ctc cgc gaa gtg gat acg aaa tgg atg gat cac    2334
   Glu Lys Val Ile Val Leu Arg Glu Val Asp Thr Lys Trp Met Asp His
               715                 720                 725
   atc gat gcg atg gat cag ctg cgg caa gga att cat ctg cgc gct tat    2382
   Ile Asp Ala Met Asp Gln Leu Arg Gln Gly Ile His Leu Arg Ala Tyr
           730                 735                 740
   gct cag aca aac ccg ctc cgc gag tat cag atg gaa ggc ttt gca atg    2430
   Ala Gln Thr Asn Pro Leu Arg Glu Tyr Gln Met Glu Gly Phe Ala Met
       745                 750                 755
   ttt gaa aac atg atc gcg gcg att gaa gat gat gta gcc aaa ttc gtc    2478
   Phe Glu Asn Met Ile Ala Ala Ile Glu Asp Asp Val Ala Lys Phe Val
   760                 765                 770                 775
   atg aag gct gaa atc gaa aac aac ctt gag cgc gaa gag gtc att caa    2526
   Met Lys Ala Glu Ile Glu Asn Asn Leu Glu Arg Glu Glu Val Ile Gln
                   780                 785                 790
   gga cag acg aca gcc cat cag ccg aaa gaa ggc gat gag gaa aaa caa    2574
   Gly Gln Thr Thr Ala His Gln Pro Lys Glu Gly Asp Glu Glu Lys Gln
               795                 800                 805
   gcg aag aaa aaa ccg gtc cgc aaa gcg gtg gat atc gga cgc aat gat    2622
   Ala Lys Lys Lys Pro Val Arg Lys Ala Val Asp Ile Gly Arg Asn Asp
           810                 815                 820
   cct tgc tac tgc gga agc gga aaa aaa tat aaa aac tgc tgc gga aga    2670
   Pro Cys Tyr Cys Gly Ser Gly Lys Lys Tyr Lys Asn Cys Cys Gly Arg
       825                 830                 835
   aca gaa taa aaagaggtgc acgcctcttt ttatttg                          2706
   Thr Glu
   840
<210> 6
   <211> 841
   <212> PRT
   <213> Bacillus licheniformis
<400> 6
   Met Leu Gly Ile Leu Asn Lys Val Phe Asp Pro Thr Lys Arg Thr Leu
   1 5 10 15
   Ser Arg Tyr Glu Lys Lys Ala Asn Glu Ile Asp Ala Leu Lys Ala Asp
   20 25 30
   Ile Glu Lys Leu Ser Asp Glu Ala Leu Lys Gln Lys Thr Ile Glu Phe
   35 40 45
   Lys Glu Arg Leu Glu Lys Gly Glu Thr Val Asp Asp Leu Leu Val Glu
   50 55 60
   Ala Phe Ala Val Val Arg Glu Ala Ser Arg Arg Val Thr Gly Met Phe
   65 70 75 80
   Pro Phe Lys Val Gln Leu Met Gly Gly Val Ala Leu His Glu Gly Asn
   85 90 95
   Ile Ala Glu Met Lys Thr Gly Glu Gly Lys Thr Leu Thr Ser Thr Met
   100 105 110
   Pro Val Tyr Leu Asn Ala Leu Ser Gly Lys Gly Val His Val Val Thr
   115 120 125
   Val Asn Glu Tyr Leu Ala Ser Arg Asp Ala Glu Glu Met Gly Lys Ile
   130 135 140
   Phe Glu Phe Leu Gly Leu Thr Val Gly Leu Asn Leu Asn Ser Leu Ser
   145 150 155 160
   Lys Asp Glu Lys Arg Glu Ala Tyr Ala Ala Asp Ile Thr Tyr Ser Thr
   165 170 175
   Asn Asn Glu Leu Gly Phe Asp Tyr Leu Arg Asp Asn Met Val Leu Tyr
   180 185 190
   Lys Glu Gln Met Val Gln Arg Pro Leu His Phe Ala Val Ile Asp Glu
   195 200 205
   Val Asp Ser Ile Leu Ile Asp Glu Ala Arg Thr Pro Leu Ile Ile Ser
   210 215 220
   Gly Gln Ala Ala Lys Ser Thr Lys Leu Tyr Val Gln Ala Asn Ala Phe
   225 230 235 240
   Val Arg Thr Leu Lys Ala Asp Gln Asp Tyr Thr Tyr Asp Val Lys Thr
   245 250 255
   Lys Gly Val Gln Leu Thr Glu Glu Gly Met Thr Lys Ala Glu Lys Ala
   260 265 270
   Phe Gly Ile Glu Asn Leu Phe Asp Val Arg His Val Ala Leu Asn His
   275 280 285
   His Ile Ala Gln Ala Leu Lys Ala His Ala Ala Met His Lys Asp Val
   290 295 300
   Asp Tyr Val Val Glu Asp Gly Gln Val Val Ile Val Asp Ser Phe Thr
   305 310 315 320
   Gly Arg Leu Met Lys Gly Arg Arg Tyr Ser Asp Gly Leu His Gln Ala
   325 330 335
   Ile Glu Ala Lys Glu Gly Leu Glu Ile Gln Asn Glu Ser Met Thr Leu
   340 345 350
   Ala Thr Ile Thr Phe Gln Asn Tyr Phe Arg Met Tyr Glu Lys Leu Ala
   355 360 365
   Gly Met Thr Gly Thr Ala Lys Thr Glu Glu Glu Glu Phe Arg Asn Ile
   370 375 380
   Tyr Asn Met Gln Val Val Thr Ile Pro Thr Asn Lys Pro Ile Ala Arg
   385 390 395 400
   Asp Asp Arg Pro Asp Leu Ile Tyr Arg Thr Met Glu Gly Lys Phe Lys
   405 410 415
   Ala Val Ala Glu Asp Val Ala Gln Arg Tyr Met Val Gly Gln Pro Val
               420                     425                     430
   Leu Val Gly Thr Val Ala Val Glu Thr Ser Glu Leu Ile Ser Arg Leu
           435                     440                     445
   Leu Lys Asn Lys Gly Ile Pro His Gln Val Leu Asn Ala Lys Asn His
       450                     455                     460
   Glu Arg Glu Ala Gln Ile Ile Glu Asp Ala Gly Gln Lys Gly Ala Val
   465                     470                     475                     480
   Thr Ile Ala Thr Asn Met Ala Gly Arg Gly Thr Asp Ile Lys Leu Gly
                   485                     490                     495
   Glu Gly Val Lys Glu Leu Gly Gly Leu Ala Val Ile Gly Thr Glu Arg
               500                     505                     510
   His Glu Ser Arg Arg Ile Asp Asn Gln Leu Arg Gly Arg Ser Gly Arg
           515                     520                     525
   Gln Gly Asp Pro Gly Ile Thr Gln Phe Tyr Leu Ser Met Glu Asp Glu
       530                     535                     540
   Leu Met Lys Arg Phe Gly Ala Glu Arg Thr Met Ala Met Leu Asp Arg
   545                     550                     555                     560
   Phe Gly Met Asp Asp Ser Thr Pro Ile Gln Ser Lys Met Val Ser Arg
                   565                     570                     575
   Ala Val Glu Ser Ser Gln Lys Arg Val Glu Gly Asn Asn Phe Asp Ala
               580                     585                     590
   Arg Lys Gln Leu Leu Gln Tyr Asp Asp Val Leu Arg Gln Gln Arg Glu
           595                     600                     605
   Val Ile Tyr Lys Gln Arg Phe Glu Val Ile Asp Ser Asp Asn Leu Arg
       610                     615                     620
   Ser Ile Val Glu Asn Met Ile Lys Ala Ser Leu Glu Arg Ala Val Ala
   625                     630                     635                     640
   Ser Tyr Thr Pro Lys Glu Asp Leu Pro Glu Glu Trp Asn Leu Asp Gly
                   645                     650                     655
   Leu Val Glu Leu Val Asn Ala Asn Phe Leu Asp Glu Gly Gly Val Glu
               660                     665                     670
   Lys Ser Asp Ile Phe Gly Lys Glu Pro Glu Glu Ile Thr Glu Leu Ile
           675                     680                     685
   Tyr Asp Arg Ile Lys Thr Lys Tyr Asp Glu Lys Glu Glu Arg Tyr Gly
       690                     695                     700
   Ser Glu Gln Met Arg Glu Phe Glu Lys Val Ile Val Leu Arg Glu Val
   705                     710                     715                     720
   Asp Thr Lys Trp Met Asp His Ile Asp Ala Met Asp Gln Leu Arg Gln
                   725                     730                     735
   Gly Ile His Leu Arg Ala Tyr Ala Gln Thr Asn Pro Leu Arg Glu Tyr
               740                     745                     750
   Gln Met Glu Gly Phe Ala Met Phe Glu Asn Met Ile Ala Ala Ile Glu
           755                     760                     765
   Asp Asp Val Ala Lys Phe Val Met Lys Ala Glu Ile Glu Asn Asn Leu
       770                     775                     780
   Glu Arg Glu Glu Val Ile Gln Gly Gln Thr Thr Ala His Gln Pro Lys
   785                     790                     795                     800
   Glu Gly Asp Glu Glu Lys Gln Ala Lys Lys Lys Pro Val Arg Lys Ala
                   805                     810                     815
   Val Asp Ile Gly Arg Asn Asp Pro Cys Tyr Cys Gly Ser Gly Lys Lys
               820                     825                     830
   Tyr Lys Asn Cys Cys Gly Arg Thr Glu
           835                     840